# EUROPÄISCHE PATENTSCHRIFT

(11) **EP 1 529 034 B1**
(45) Veröffentlichungstag und Bekanntmachung des Hinweises auf die Patenterteilung: **20.02.2008**
(21) Anmeldenummer: 03784050.1
(22) Anmeldetag: 23.07.2003
(51) Int. Cl.: C07D 231/20, C07D 403/12, A01N 43/56

(54) **3-AMINOCARBONYL SUBSTITUIERTE BENZOYLPYRAZOLONE**
BENZOYLPYRAZOLONES SUBSTITUTED BY 3-AMINOCARBONYL
BENZOYLPYRAZOLONES SUBSTITUEES PAR 3-AMINOCARBONYLE

(30) Priorität: 06.08.2002 DE 10235945
(43) Veröffentlichungstag der Anmeldung: 11.05.2005
(73) Patentinhaber: Bayer CropScience AG, 40789 Monheim (DE)
(72) Erfinder: SEITZ, Thomas, 68519 Viernheim (DE); VAN ALMSICK, Andreas, 61184 Karben (DE); WILLMS, Lothar, 65719 Hofheim (DE); AULER, Thomas, Dr., 42799 Leichlingen (DE); BIERINGER, Hermann, 65817 Eppstein (DE); MENNE, Hubert, 55252 Mainz-Kastel (DE)
(86) Internationale Anmeldenummer: PCT/EP2003/008047
(87) Internationale Veröffentlichungsnummer: WO 2004/014863

(56) Entgegenhaltungen:
- EP-A- 0 282 944
- EP-A- 0 352 543
- WO-A-97/46530
- DATABASE WPI Section Ch, Week 200002 Derwent Publications Ltd., London, GB; Class C02, AN 2000-018692 XP002260342 & JP 11 292849 A (NIPPON SODA CO), 26. Oktober 1999 (1999-10-26)

## Beschreibung

Die Erfindung betrifft das technische Gebiet der Herbizide, insbesondere das der Herbizide aus der Gruppe der Benzoylpyrazolone zur selektiven Bekämpfung von Unkräutern und Ungräsern in Nutzpflanzenkulturen, insbesondere in Reiskulturen.

Aus verschiedenen Schriften ist bereits bekannt, daß bestimmte Benzoylpyrazolone herbizide Eigenschaften besitzen. So sind aus EP-A 0 352 543 Benzoylpyrazolone bekannt, die in 3-Position des Phenylrings unter anderem einen über ein Sauerstoffatom gebundenen Alkoxycarbonylalkyl-, einen Aminoalkyl- oder einen Alkylcarbonyl-Rest tragen. WO 97/41106 beschreibt Benzoylpyrazolone, die in gleicher Position beispielsweise einen Alkylaminosulfonyl- oder Alkylsulfonylamino-Rest tragen. Die aus diesen Schriften bekannten Verbindungen zeigen jedoch häufig eine nicht ausreichende herbizide Wirksamkeit.

Aufgabe der vorliegenden Erfindung ist die Bereitstellung von herbizid wirksamen Verbindungen mit - gegenüber den im Stand der Technik offenbarten Verbindungen - verbesserten herbiziden Eigenschaften.

Es wurde nun gefunden, daß Benzoylpyrazolone, die in 3-Position des Phenylrings einen über ein Atom aus der Gruppe Sauerstoff, Stickstoff und Schwefel gebundenen Aminocarbonylalkyl-Rest tragen, als Herbizide besonders gut geeignet sind. Ein Gegenstand der vorliegenden Erfindung sind daher Verbindungen der Formel (I) oder deren Salze 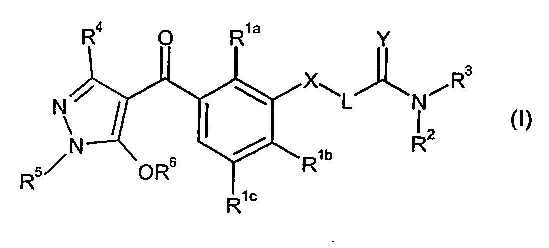 worin die Reste und Indizes folgende Bedeutungen haben:
- X: bedeutet O, S(O)ₙ, N-H oder N-R²;
- L: bedeutet eine geradkettige oder verzweigte durch w Reste aus der Gruppe Halogen, Cyano und Nitro und durch v Reste R² substituierte (C₁-C₆)-Alkylen-, (C₂-C₆)-Alkenylen- oder (C₂-C₆)-Alkinylenkette;
- Y: bedeutet Sauerstoff oder Schwefel;
- R^{1a}, R^{1b}, R^{1c}: bedeuten unabhängig voneinander Wasserstoff, Mercapto, Nitro, Halogen, Cyano, Thiocyanato, (C₁-C₆)-Alkyl-CO-O, (C₁-C₆)-Alkyl-S(O)ₙ-O, (C₁-C₆)-Alkyl-S(O)ₘ, (C₁-C₆)-Haloalkyl-S(O)ₘ, (C₃-C₇)-Cycloalkyl-S(O)ₘ, Di-(C₁-C₆)-Alkyl-N-SO₂, (C₁-C₆)-Alkyl-SO₂-NH, (C₁-C₆)-Alkyl-NH-CO, Di-(C₁-C₆)-Alkyl-N-CO, (C₁-C₆)-Alkyl-SO₂-[(C₁-C₆)-alkyl]amino, (C₁-C₆)-Alkyl-CO-[(C₁-C₆)-alkyl]amino, (C₁-C₆)-Alkyl-O-CH₂, (C₁-C₆)-Alkyl-S(O)ₙ-CH₂, (C₁-C₆)-Alkyl-NH-CH₂, 1,2,4-Triazol-1-yl, 1,2,4-Triazol-1-yl-CH₂, jeweils durch v Reste aus der Gruppe Cyano, Nitro und Halogen substituiertes (C₁-C₆)-Alkyl-(Y)ₚ, (C₂-C₆)-Alkenyl-(Y)ₚ, (C₂-C₆)-Alkinyl-(Y)ₚ, (C₃-C₉)-Cycloalkyl-(Y)ₚ, (C₃-C₉)-Cyloalkenyl-(Y)ₚ, (C₁-C₆)-Alkyl-(C₃-C₉)-cycloalkyl-(Y)ₚ oder (C₁-C₆)-Alkyl-(C₃-C₉)-cycloalkenyl-(Y)ₚ;
- R², R³: bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉)-cycloalkyl, (C₁-C₆)-Alkyl-(C₃-C₉)-cycloalkenyl, (C₂-C₆)-Alkenyl-(C₃-C₉)-cycloalkyl, (C₂-C₆)-Alkenyl-(C₃-C₉)-cycloalkenyl, (C₂-C₆)-Alkinyl-(C₃-C₉)-cycloalkyl, (C₂-C₆)-Alkinyl-(C₃-C₉)-cycloalkenyl, geradkettiges oder verzweigtes [O-C(R⁶)₂]_{w}-[O-C(R⁶)-₂]ₓ-R⁶, (C₁-C₆)-Alkyl-aryl, (C₂-C₆)-Alkenyl-aryl, (C₂-C₆)-Alkinyl-aryl, geradkettiges oder verzweigtes [O-C(R⁶)₂]_{w}-[O-C(R⁶)₂]ₓ-Aryl, wobei die letzten 16 der vorstehend genannten Reste durch v Reste aus der Gruppe Cyano, Nitro und Halogen substituiert sind,
jeweils durch v Reste aus der Gruppe Cyano, Nitro, Halogen, (C₁-C₆)-Alkyl-(Y)ₚ und Halogen-(C₁-C₆)-alkyl-(Y)ₚ substituiertes Aryl, Heterocyclyl oder Heteroaryl,
oder
- R² und R³: bilden gemeinsam mit dem sie bindenden Stickstoffatom einen durch jeweils v Reste aus der Gruppe Cyano, Nitro, Halogen, (C₁-C₆)-Alkyl-(Y)ₚ und Halogen-(C₁-C₆)-alkyl-(Y)ₚ substituierten 5- oder 6-gliedrigen, gesättigten, teilweise oder vollständig ungesättigten Ring, der n Heteroatome aus der Gruppe Sauerstoff und Stickstoff enthält,
oder
- R² und R³: bilden gemeinsam mit dem sie bindenden Stickstoffatom einen durch v Reste aus der Gruppe Cyano, Nitro, Halogen, (C₁-C₆)-Alkyl-(Y)ₚ und Halogen-(C₁-C₆)-alkyl-(Y)ₚ substituierten Ring aus der Gruppe Benzothiazol, Benzoxazol, Benzopyrazol und Benzopyrrol;
- R⁴: bedeutet Wasserstoff, (C₁-C₆)-Alkyl oder (C₁-C₆)-Halogenalkyl, (C₃-C₉)-Cycloalkyl oder (C₃-C₉)-Halogencycloalkyl;
- R⁵: bedeutet (C₁-C₆)-Alkyl, Halogen-(C₁-C₆)-alkyl, (C₃-C₉)-Cycloalkyl, (C₃-C₉)-Halogen-cycloalkyl, oder jeweils durch v Reste aus der Gruppe Halogen, Nitro, Cyano, (C₁-C₄)-Alkyl, Halogen-(C₁-C₄)-alkyl, (C₁-C₄)-Alkoxy und Halogen-(C₁-C₄)-alkoxy substituiertes Phenyl;
- R⁶: bedeutet Wasserstoff, (C₁-C₆)-Alkyl, Halogen-(C₁-C₆)-alkyl, (C₁-C₆)-Alkylcarbonyl, Halogen-(C₁-C₆)-alkylcarbonyl, (C₁-C₆)-Alkoxycarbonyl Halogen-(C₁-C₆)-alkoxycarbonyl, (C₁-C₆)-Alkylaminocarbonyl, Halogen-(C₁-C₆)-alkylaminocarbonyl, (C₁-C₆)-Dialkylaminocarbonyl, Halogen-(C₁-C₆)-dialkylaminocarbonyl, (C₁-C₆)-Alkylsulfonyl, Halogen-(C₁-C₆)-alkylsulfonyl, jeweils durch v Reste aus der Gruppe Halogen, Nitro, Cyano, (C₁-C₄)-Alkyl, Halogen-(C₁-C₄)-alkyl, (C₁-C₄)-Alkoxy und Halogen-(C₁-C₄)-alkoxy substituiertes Benzyl, Benzoyl, Benzoylmethyl, Phenoxycarbonyl oder Phenylsulfonyl;
- m: bedeutet 1 oder 2;
- n: bedeutet 0, 1 oder 2;
- p: bedeutet 0 oder 1;
- v: bedeutet 0, 1, 2 oder 3;
- w und x: bedeuten jeweils unabhängig voneinander 0,1, 2, 3 oder 4;
wobei w und x nicht gleichzeitig null sein sollen.

Für den Fall, daß R⁶ für Wasserstoff steht, können die erfindungsgemäßen Verbindungen der Formel (I) in Abhängigkeit von äußeren Bedingungen, wie Lösungsmittel und pH-Wert, in unterschiedlichen tautomeren Strukturen auftreten. Je nach Art der Substituenten enthalten die Verbindungen der Formel (I) ein acides Proton, das durch Umsetzung mit einer Base entfernt werden kann. Als Basen eignen sich beispielsweise Hydride, Hydroxide und Carbonate von Alkali- und Erdalkalimetallen, wie Lithium, Natrium, Kalium, Magnesium und Calcium, sowie Ammoniak und organische Amine wie Triethylamin und Pyridin. Solche Salze sind ebenfalls Gegenstand der Erfindung.

In Formel (I) und allen nachfolgenden Formeln können Alkylreste mit mehr als zwei-C-Atomen geradkettig oder verzweigt sein. Alkylreste bedeuten z.B. Methyl, Ethyl, n- oder i-Propyl, n-, i-, t- oder 2,Butyl, Pentyle, Hexyle, wie n-Hexyl, i-Hexyl und 1,3-Dimethylbutyl. Die Kohlenstoffkette L kann ebenfalls, je nach Anzahl ihrer C-Atome geradkettig oder verzweigt sein. Die daran gebundenen Reste können sich an beliebiger Position dieser Kette befinden.

Ist eine Gruppe mehrfach durch Reste substituiert, so ist darunter zu verstehen, daß diese Gruppe durch ein oder mehrere gleiche oder verschiedene der genannten Reste substituiert ist.

Cycloalkyl bedeutet ein carbocyclisches, gesättigtes Ringsystem mit drei bis neun C-Atomen, z.B. Cyclopropyl, Cyclopentyl oder Cyclohexyl. Analog bedeutet Cycloalkenyl eine monocyclische Alkenylgruppe mit drei bis neun Kohlenstoffringgliedern, z.B. Cyclopropenyl, Cyclobutenyl, Cyclopentyl und Cyclohexenyl, wobei sich die Doppelbindung an beliebiger Position befinden kann.

im Falle zusammengesetzter Reste, wie Cycloalkylalkenyl, kann sich der erstgenannte Rest an beliebiger Position des zweitgenannten befinden.

Im Falle einer zweifach substituierten Aminogruppe, wie Dialkylamino, können diese beiden Substituenten gleich oder verschieden sein.

Halogen bedeutet Fluor, Chlor, Brom oder Iod. Halogenalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Halogenalkoxy ist z.B. OCF₃, OCHF₂, OCH₂F, CF₃CF₂O, OCH₂CF₃ und OCH₂CH₂Cl; entsprechendes gilt für Halogenalkenyl und andere durch Halogen substituierte Reste.

Unter dem Begriff Heterocyclyl sind drei- bis sechsgliedrige, gesättigte oder partiell ungesättige mono- oder polycyclische Heterocyclen zu verstehen, die ein bis drei Heteroatome ausgewählt aus einer Gruppe bestehend aus Sauerstoff, Stickstoff und Schwefel enthalten. Die Verknüpfung kann, sofern chemisch möglich an beliebiger Position des Heterocyclus erfolgen. Beispiele dafür sind Oxiranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-isoxazoldinyl, 4-Isoxazolidinyl, 5-lsoxoazolidinyl, 3-Isothioazolidinyl, 4-Isothiazolidinyl, 5-Isothiazolidinyl, 1-Pyrazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxa-diazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydrofur-4-yl, 2,3-Dihydrofur-5-yl, 2,5-Dihydrofur-2-yl, 2,5-Dihydrofur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2.,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydro-isoxazol-4-yl, 4,5-Dihydroisoxazol-5-yl, 2,3-Dihydroisothiazol-3-yl, 2,3-Dihydroisothiazol-4-yl, 2,3-Dihydroisothiazol-5-yl, 2,5-Dihydroisoxazol-3-yl, 2,5-Dihydroisoxazol-4-yl, 2,5-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 1-Morpholinyl, 2-Morpholinyl, 3-Morpholinyl, 1-Piperidinyl, 2-Piperidinyl, 3-Piperidinyl, 4-Piperidinyl, 3-Tetrahydropyridazinyl, 4-Tetrahydro-pyridazinyl, 2-Tetrahydropyrimidinyl, 4-Tetrahydropyrimidinyl, 5-Tetrahydro-pyrimidinyl, 2-Tetrahydropyrazinyl, 1,3,5-Tetrahydrotriazin-2-yl, 1,2,4-Tetrahydro-triazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,3-Dithian-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl.

Aryl steht für einen aromatischen mono- oder polycyclischen Kohlenwasserstoffrest, z.B. Phenyl, Naphthyl, Biphenyl und Phenanthryl, vorzugsweise Phenyl. Die Verknüpfung kann grundsätzlich an beliebiger Position von Aryl erfolgen.

Heteroaryl steht für einen aromatischen mono-, bi- oder tricyclischen Rest, der neben Kohlenstoffringgliedern ein bis vier Stickstoffatome oder ein bis drei Stickstoffatome und ein Sauerstoff- oder ein Schwefelatom oder ein Sauerstoff- oder ein Schwefelatom enthält. Die Verknüpfung kann, sofern chemisch möglich, an beliebiger Position von Aryl erfolgen. Beispiele für 5-gliedriges Heteroaryl sind 2-Pyrrolyl, 3-Pyrrolyl, 1-Pyrazolyl, 3-Pyrazolyl, 4-Pyrazolyl, 5-Pyrazolyl, 2-Imidazolyl, 4-Imidazolyl, 1,2,4-Triazolyl-3-yl, 1,3,4-Triazol-2-yl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl. Beispiele für 6-gliedriges Heteroaryl sind 2-Pyridinyl, 3-Pyridinyl, 4-Pyridinyl, 3-Pyridazinyl, 4-Pyridazinyl, 2-Pyrimdinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl. Beispiele für anneliertes 5-gliedriges Heteroaryl sind Benzothiazol-2-yl und Benzoxazol-2-yl. Beispiele für benzokondensierte 6-gliedriges Heteroaryl sind Chinolin, Isochinolin, Chinazolin und Chinoxalin.

Ist eine Gruppe mehrfach substituiert, so ist darunter zu verstehen, daß bei der Kombination der verschiedenen Substituenten die allgemeinen Grundsätze des Aufbaus chemischer Verbindungen zu beachten sind, d.h. daß nicht Verbindungen gebildet werden, von denen der Fachmann weiß, daß sie chemisch instabil oder nicht möglich sind.

Die Verbindungen der Formel (I) können je nach Art und Verknüpfung der Substituenten als Stereoisomere vorliegen. Sind beispielsweise ein oder mehrere asymmetrische C-Atome vorhanden, so können Enantiomere und Diastereomere auftreten. Stereoisomere lassen sich aus den bei der Herstellung anfallenden Gemischen nach üblichen Trennmethoden, z.B. durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Als vorteilhaft haben sich Verbindungen der Formel (I) herausgestellt, worin R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉)-cycloalkyl, (C₁-C₆)-Alkyl-(C₃-C₉)-cycloalkenyl, (C₂-C₆)-Alkenyl-(C₃-C₉)-cycloalkyl, (C₂-C₆)-Alkenyl-(C₃-C₉)-cycloalkenyl, (C₂-C₆)-Alkinyl-(C₃-C₉)-cycloalkyl, (C₂-C₆)-Alkinyl-(C₃,C₉),cycloalkenyl, geradkettiges oder verzweigtes [O-C(R⁶)₂]_{w}-[O-C(R⁶)-₂]ₓ-R⁶, (C₁-C₆)-Alkyl-aryl, (C₂-C₆)-Alkenyl-aryl, (C₂-C₆)-Alkinyl-aryl, geradkettiges oder verzweigtes [O-C(R⁶)₂]_{w}-[O-C(R⁶)₂]ₓ-Aryl, wobei die letzten 16 der vorstehend genannten Reste durch v Reste aus der Gruppe Cyano, Nitro und Halogen substituiert sind,
durch v Reste aus der Gruppe Cyano, Nitro, Halogen, (C₁-C₆)-Alkyl-(Y)ₚ und Halogen-(C₁-C₆)-alkyl-(Y)ₚ substituiertes Aryl,
oder
R² und R³ bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten 5- oder 6-gliedrigen, gesättigten, teilweise oder vollständig ungesättigten Ring, der n Heteroatome aus der Gruppe Sauerstoff und Stickstoff enthält,
oder
R² und R³ bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten Ring aus der Gruppe Benzothiazol, Benzoxazol, Benzopyrazol und Benzopyrrol, und die anderen Substituenten und Indices jeweils die weiter oben genannten Bedeutungen haben.

Bevorzugt sind Verbindungen der allgemeinen Formel (I), worin Y für Sauerstoff und R^{1c} für Wasserstoff stehen, und die anderen Substituenten und Indices jeweils die weiter oben genannten Bedeutungen haben.

Bevorzugt sind auch Verbindungen der allgemeinen Formel (I), worin
- X: O oder S(O)ₙ bedeutet;
- R^{1a}, R^{1b}: unabhängig voneinander F, Cl, Br, CH₃, CH₃S, CH₃O, CH₃SO₂, C₂H₅SO₂, CF₃CH₂SO₂, Cyclopropyl-SO₂, CF₃ oder NO₂ bedeuten;
- R², R³: unabhängig voneinander Wasserstoff, (C₁-C₆)-Alkyl, (C₂-C₆)-Alkenyl, (C₂-C₆)-Alkinyl, (C₃-C₉)-Cycloalkyl, (C₁-C₆)-Alkyl-(C₃-C₉)-cycloalkyl, wobei die letzten 5 genannten Reste mit v Resten aus der Gruppe Cyano, Nitro und Halogen substituiert sind, Aryl, (C₁-C₆)-Alkyl-aryl, wobei die letzten 2 genannten Reste mit v Resten aus der Gruppe Cyano, Nitro, Halogen, (C₁-C₆)-Alkyl-(Y)ₚ und Halo-(C₁-C₆)-alkyl-(Y)ₚ substituiert sind, bedeuten, oder
- R² und R³: bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten 5- oder 6-gliedrigen, gesättigten, teilweise oder vollständig ungesättigten Ring, der n Heteroatome aus der Gruppe Sauerstoff und Stickstoff enthält,
oder
- R² und R³: bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten Ring aus der Gruppe Benzothiazol, Benzoxazol, Benzopyrazol und Benzopyrrol, und die anderen Substituenten und Indices jeweils die weiter oben genannten Bedeutungen haben.

Besonders bevorzugt sind Verbindungen der allgemeinen Formel (I), worin X für Sauerstoff steht, und die anderen Substituenten und Indices jeweils die weiter oben genannten Bedeutungen haben.

Ebenfalls bevorzugt sind Verbindungen der allgemeinen Formel (I), worin
- R², R³: unabhängig voneinander Wasserstoff oder (C₁-C₆)-Alkyl bedeuten,
oder
- R² und R³: bilden gemeinsam mit dem sie bindenden Stickstoffatom einen Ring aus der Gruppe Morpholin, Pyrrolidin, Piperidin, Pyrrol, Pyrazol und 2,3-Dihydroindol;
- R⁴: für Wasserstoff, Methyl oder Cyclopropyl steht, und die anderen Substituenten und Indices jeweils die weiter oben genannten Bedeutungen haben.

Weiterhin besonders bevorzugt sind Verbindungen der allgemeinen Formel (I), worin
- R⁶: Wasserstoff, (C₁-C₆)-Alkyl, (C₁-C₆)-Alkylcarbonyl, (C₁-C₆)-Alkylsulfonyl, jeweils durch v Reste aus der Gruppe Halogen, Nitro, Cyano, (C₁-C₄)-Alkyl, Halogen-(C₁-C₄)-alkyl, (C₁-C₄)-Alkoxy und Halogen-(C₁-C₄)-alkoxy substituiertes Benzoyl oder Phenylsulfonyl, und die anderen Substituenten und Indices jeweils die weiter oben genannten Bedeutungen haben.

Ganz besonders bevorzugt sind Verbindungen der allgemeinen Formel (I), worin
- L: CH₂, C(CH₃)H oder CH₂CH₂ bedeutet;
- R^{1a}, R^{1b}: unabhängig voeinander Cl, Br, NO₂, CH₃, CH₃SO₂ oder C₂H₅SO₂ bedeuten;
- R², R³: jeweils Wasserstoff oder (C₁-C₆)-Alkyl bedeuten;
- R⁵: Methyl oder Ethyl bedeutet;
und die anderen Substituenten und Indices jeweils die weiter oben genannten Bedeutungen haben.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R⁶ für Wasserstoff steht, können beispielsweise nach der in Schema 1 angegebenen Methode durch basenkatalysierte Umsetzung einer Verbindung der Formel (IIIa), in der T für Halogen, Hydroxy oder Alkoxy steht, mit einem Pyrazol (II) in Anwesenheit einer Cyanid-Quelle hergestellt werden. Solche Methoden sind beispielsweise in WO 99/10328 beschrieben.

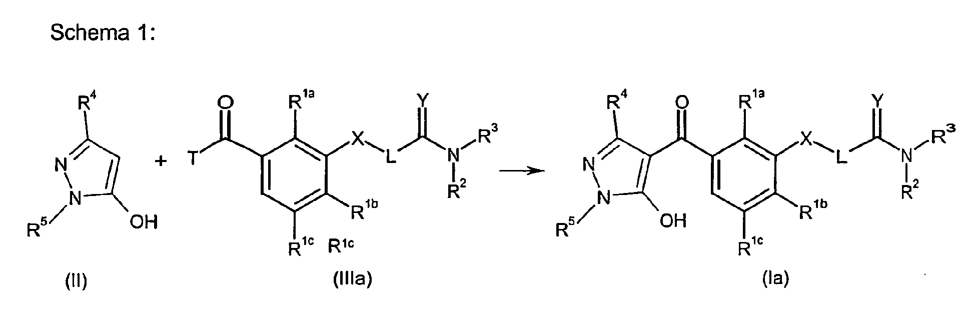

Verbindungen der Formel (IIIa) können beispielsweise nach Schema 2 aus Verbindungen der Formel (IIIb) und (IVa), in der E für eine Fluchtgruppe wie Halogen, Mesyl, Tosyl oder Triflat steht, gemäß an sich bekannten Methoden hergestellt werden. Solche Methoden sind z.B. aus Houben-Weyl Band 6/3, S. 54 bis 69, Band 9, S. 103 bis 115 und Band 11, S. 97 bekannt.

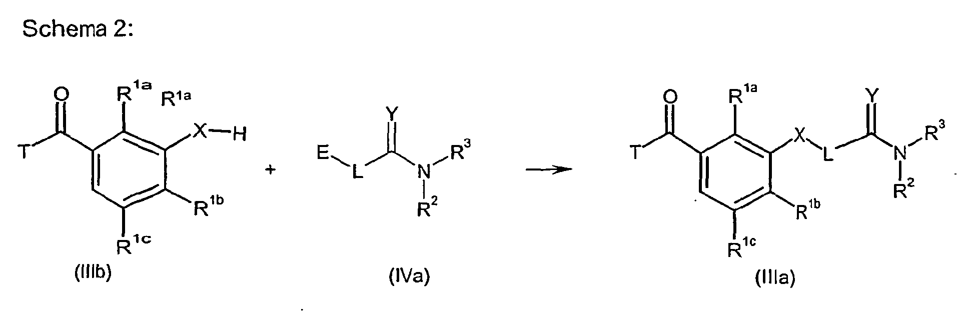

Verbindungen der Formel (IIIa) können gemäß Schema 3 auch durch Umsetzung von Verbindungen der Formel (IIIc), worin E¹ für eine Fluchtgruppe wie Triflat oder Nonaflat steht, mit Verbindungen der Formel (IVb) hergestellt werden. Solche Methoden sind z. B. aus WO 98/42648, Houben-Weyl Band 6/3, S. 75 bis 78, Band 9, S. 103 bis 105 bekannt.

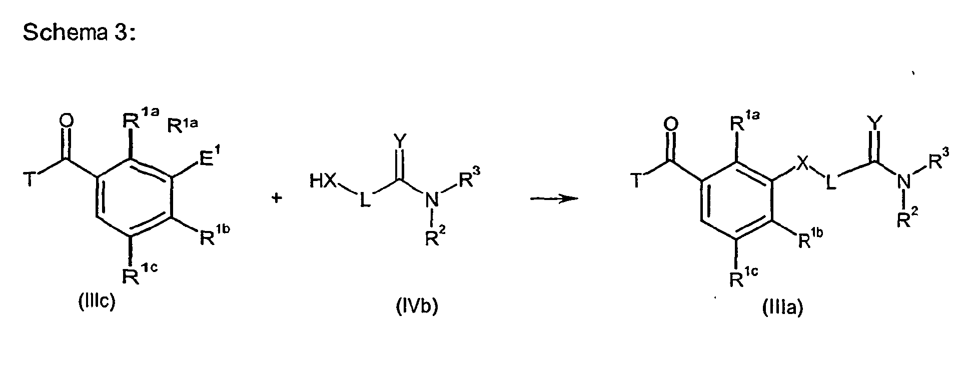

Ebenso können Verbindungen der Formel (IIIa) gemäß Schema 4 auch durch Umsetzung von Verbindungen der Formel (IIId) mit Verbindungen E²-R³, worin E² für eine Abgangsgruppe wie Chlor, Brom oder Mesyl steht hergestellt werden. Solche Methoden sind z. B. aus Houben-Weyl Band 8, S. 708, E5/2, S.998 und 1213 bekannt.

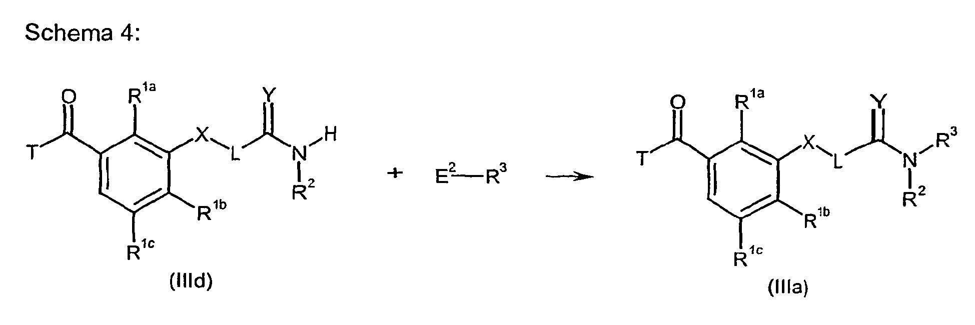

Verbindungen der Formel (IVa) können beispielsweise nach Schema 5 aus Verbindungen der Formel (VII), in der E für eine Gruppe wie Chlor oder Alkoxy und E² für eine Gruppe wie Chlor, Brom, Mesyl oder Tosyl steht, mit Aminen der Formel (VIII) gemäß an sich bekannten Methoden hergestellt werden. Solche Methoden sind z.B. aus Houben-Weyl Band 8, S. 647 bis 660, Band 11/2, S. 1 bis 73 (insbesondere S. 10 bis 14 und 20 bis 23), Band E 5/2, S. 934 bis 1135 und aus J. Org. Chem. 39 (1974) S. 2607 bis 2612 bekannt.

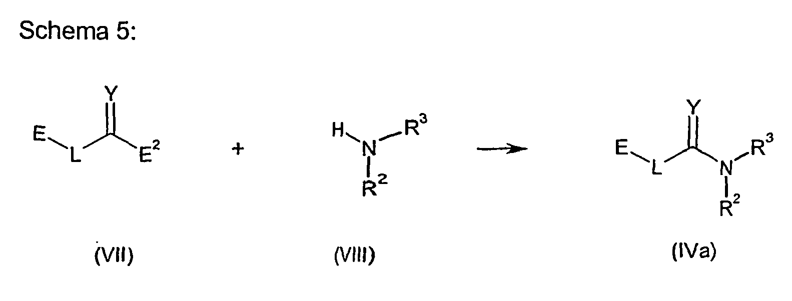

Verbindungen der Formel (IVb) können beispielsweise nach den in US 4,264,520, DE 3 222 229 und J. Med. Chem. 39 (1996) 26, S. 5236 bis 5245 beschriebenen Methoden hergestellt werden.

Erfindungsgemäße Verbindungen der Formel (I), in der R⁶ für andere Reste als Hydroxy steht, können beispielsweise gemäß Schema 6 durch dem Fachmann an sich bekannte Substitutionsreaktionen hergestellt werden. Dazu werden Verbindungen der Formel (Ia) mit Verbindungen der Formel (VIII), in der E³ für eine nucleophil austauschbare Abgangsgruppe steht, umgesetzt. Solche Methoden sind z. B. aus WO 99/10328 bekannt.

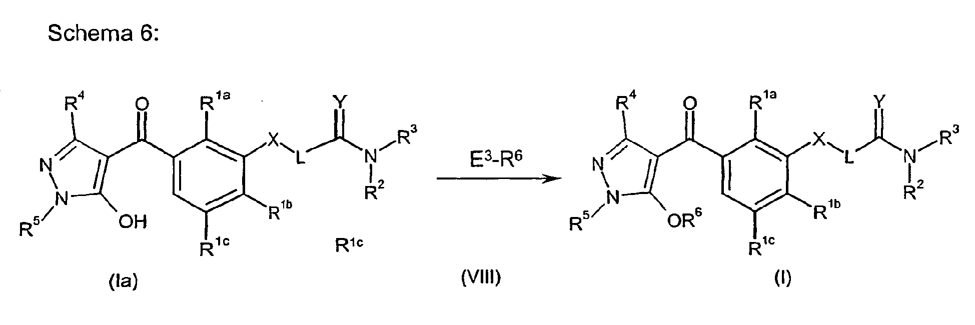

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Amaranthus retroflexus, Avena sp., Echinochloa sp., Cyperus serotinus, Lolium multiflorum, Setaria viridis, Sagittaria pygmaea, Scirpus juncoides, Sinapis sp. und Stellaria media.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Weizen, Mais und Reis auf. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment-der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vor-gegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können z.B. verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäure-ester oder Polyoxethylensorbitanester wie z.B. Polyoxyethylen-sorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weisegewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seilen 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl, chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; mesotrione; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; suclotrione; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1 H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-92O1; ET-751; KIH-6127 und KIH-2O23.

Zur Anwendung werden die in handelsüblicher Form vorliegenden Formulierungen in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

Die Herstellung der Ausgangsverbindung 2,4-Dibrom-3-hydroxybenzoesäureethylester erfolgte gemäß US 5,026,896.
Die Abkürzung RT steht für Raumtemperatur. R^{f} bedeutet Retentionswert.

### Herstellung von 1-Methyl-4-(2-chlor-3-(pyrrolidinocarbonyl-methoxy)-4-ethylsulfonylbenzoyl)-pyrazolon (Tabellenbeispiel Nr. 1.60)

### Schritt 1: 2-Chlor-3-hydroxy-4-ethylsulfonyl-benzoesäuremethylester

33.0 g 2-Chlor-3-hydroxy-4-ethylsulfonyl-benzoesäure wurden in 1300 ml MeOH gelöst. Es wurden 174 ml konz. H₂SO₄ zugetropft und dann 5 h unter Rückfuß erhitzt. Die Mischung wurde eingeengt, und der Rückstand wurde in CH₂Cl₂ aufgenommen. Es wurde mit Wasser gewaschen, über Na₂SO₄ getrocknet und vollständig eingeengt. Man erhielt 2-Chlor-3-hydroxy-4-ethylsulfonylbenzoesäuremethylester als gelbes, zähes Öl.

| | | |
|---|---|---|
| Ausbeute: | 28.23 g (81 % der Theorie) | R^{f}: (Essigester) 0.45 |
| ¹H-NMR: | δ [CDCl₃] 1.32 (t, 3H), 3.24 (q, 2H), 3.96 (s, 3H), 7.38 (d, 1H), 7.65 (d, 1H) | |

### Schritt 2: 2-Chlor-3-(pyrrolidinocarbonyl-methoxy)-4-ethylsulfonyl-benzoesäuremethylester

0.595 g K₂CO₃, 0.107g Kl und 0.459 g Chloracetyfpyrrolidid wurden in 30 ml Aceton vorgelegt. Bei RT wurden 0.600 g 2-Chlor-3-hydroxy-4-ethylsulfonyl-benzoesäuremethylester zugegeben und für 4 h unter Rückfluß erhitzt. Dann wurde auf Wasser gegeben und mit Essigester extrahiert. Die organischen Phasen wurden mit Wasser gewaschen, über Na₂SO₄ getrocknet und eingeengt. Chromatographie an Kieselgel (Laufmittel: n-Heptan/Essigester) ergab 2-Chlor-3-(pyrrolidinocarbonyl-methoxy)-4-ethylsulfonyl-benzoesäuremethylester als farbloses, zähes Öl.

| | |
|---|---|
| Ausbeute: | 0.50 g (58% der Theorie) |
| ¹H-NMR: | δ [CDCl₃] 1.24 (t, 3H), 1.88 (m, 2H), 2.00 (m, 2H), 3.29 (m, 2H), 3.57 (m, 4H), 3.69 (q, 2H), 4.00 (s, 3H), 4.82 (s, 2H), 7.69 (d, 1H), 7.93 (d, 1H). |

### Schritt 3: 2-Chlor-3-(pyrrolidinocarbonyl-methoxy)-4-ethylsulfonyl-benzoesäure

0.500 g 2-Chlor-3-(pyrrolidinocarbonyl-methoxy)-4-ethylsulfonyl-benzoesäuremethylester wurden in 20 ml THF und 20 ml Wasser gelöst und mit 0.056 g NaOH versetzt und 12 h bei RT gerührt. Die Mischung wurde mit 6 N HCl versetzt und mit CH₂Cl₂ extrahiert. Trocknen der organischen Phase mit Na₂SO₄ ergab 2-Chlor-3-(pyrrolidinocarbonyl-methoxy)-4-ethylsulfonyl-benzoesäure als farbloses, zähes Öl.

| | |
|---|---|
| Ausbeute: | 0.42 g (87% der Theorie) |
| ¹H-NMR: | δ [CDCl₃] 1.22 (t, 3H), 1.91 (m, 2H), 2.00 (m, 2H), 3.32 (m, 2H), 3.60 (m, 4H), 4.85 (s, 2H), 7.77 (d, 1H), 7.91 (d, 1H). |

### Schritt 4: (5-(1-Methyl-pyrazolyl))-(2-Chlor-3-(pyrrolidinocarbonyl-methoxy)-4-ethylsulfonyl)-benzoat

0.210 g (0.60 mmol) 2-Chlor-3-(pyrrolidinocarbonyl-methoxy)-4-ethylsulfonyl-benzoesäure, 0.092 g 1-Methylpyrazolon, 0.109 g N'-(3-Dimethylaminopropyl)-N-ethylcarbodiimid Hydrochlorid und 0.001 g DMAP wurden in 15 ml CH₂Cl₂ 3 h bei RT gerührt. Anschliessend mit 0.5 N HCl angesäuert und die Phasen wurden getrennt. Die wäßrige Phase wurde mit CH₂Cl₂ extrahiert. Nach Trocknen der organischen Phasen über Na₂SO₄ und Einengen wurde (5-(1-Methyl-pyrazolyl))-(2-Chlor-3-(pyrrolidinocarbonyl-methoxy)-4-ethylsulfonyl)-benzoat in Form eines braunen Harzes erhalten, das für die Folgeumsetzung ausreichend rein war.

| | |
|---|---|
| Ausbeute: | 0.210 g |
| ¹H-NMR: | δ [CDCl₃] 1.26 (t, 3H), 1.89 (m, 2H), 2.00 (m, 2H), 3.28 (m, 2H), 3.58 (m, 2H), 3,66 (q, 2H), 3.75 (s, 3H), 4.83 (s, 2H), 6.15 (s, 1H), 7.41 (s, 1H), 7.79 (d, 1H), 7.96 (d, 1H). |

### Schritt 5: 1 -Methyl-4-(2-chlor-3-(pyrrolidinocarbonyl-methoxy)-4-ethylsulfonyl-benzoyl)-pyrazolon

0.210 g (5-(1-Methyl-pyrazolyl))-(2-Chlor-3-(pyrrolidinocarbonyl-methoxy)-4-ethylsulfonyl)-benzoat wurden in 10 ml Acetonitril gelöst. Es wurden 3 Tropfen Acetoncyanhydrin, 0.079 g NEt₃, sowie 0.009 g KCN zugegeben. Nach 3 h Rühren bei RT wurde mit Wasser verdünnt, mit 0.5 N HCl angesäuert und mit CH₂Cl₂ extrahiert. Nach Trocknen über Na₂SO₄, Einengen und Chromatographie an reversed-phase-Kieselgel (Laufmittel: Acetonitril/Wasser-Gradient) erhielt man 1-Methyl-4-(2-chlor-3-(pyrrolidinocarbonyl-methoxy)-4-ethylsulfonyl-benzoyl)-pyrazolon als farbloses, zähes Öl.

| | | |
|---|---|---|
| Ausbeute: | 0.036 g (ca. 11 % der Theorie) | R^{f} (Essigester): 0.01 |
| ¹H-NMR: | δ [CDCl₃] 1.28 (t, 3H), 1.91 (m, 2H), 2.00 (m, 2H), 3.31 (m, 2H), 3.59 (m, 2H), 3,69 (q, 2H), 3.73 (s, 3H), 4.85 (s, 2H), 7.35 (s, 1H), 7.41 (d, 1H), 8.00 (d, 1H). | |

### Herstellung von (1,3-Dimethyl-4-(2,4-dibrom-3-(N,N-di-n-propylaminocarbonyl-methoxy)-benzoyl)-pyrazolon (Tabellenbeispiel Nr. 3.34)

### Schritt 1: 2,4-Dibrom-3-(N,N-d i-n-propylaminocarbonyl-methoxy)-benzoesäureethylester

0.853 g K₂CO₃, 0.154g KI und 1.000 g 2,4-Dibrom-3-hydroxy-benzoesäureethylester wurden in 10 ml Aceton vorgelegt. Bei RT wurden 0.783 g N,N-di-n-Propyl-chloracetamid zugegeben. Dann wurde für 4 h unter Rückfluß erhitzt. Anschliessend wurde auf Wasser gegeben und mit Diisopropylether extrahiert. Die organischen Phasen wurden mit Wasser gewaschen, über Na₂SO₄ getrocknet und eingeengt. Chromatographie an Kieselgel (Laufmittel: n-Heptan/Essigester) ergab 2,4-Dibrom-3-(N,N-di-n-propylaminocarbonyl-methoxy)-benzoesäureethylester als braunes Öl.

| | |
|---|---|
| Ausbeute: | 1.27 g (83% der Theorie) |
| ¹H-NMR: | δ [CDCl₃] 0.93 (m, 6H), 1.20 (t, 3H), 1,64 (m, 4H), 3.32 (m,4H), 4.20 (q, 4H), 4.66 (s, 2H), 7.40 (d, 1H), 7.57 (d, 1H). |

### Schritt 2: 2,4-Dibrom-3-(N,N-di-n-propylaminocarbonyl-methoxy)-benzoesäure

1.24 g (2.70 mmol) 2,4-Dibrom-3-(N,N-di-n-propylaminocarbonyl-methoxy)-benzoesäureethylester wurden in 10 ml THF und 10 ml H₂O gelöst und mit 0.117 g NaOH versetzt. Nach 12 h Rühren bei RT wurde die Mischung mit 6 N HCl versetzt und mit CH₂Cl₂ extrahiert. Trocknen der organischen Phase mit Na₂SO₄ ergab 2,4-Dibrom-3-(N,N-di-n-propylaminocarbonyl-methoxy)-benzoesäure als eines farbloses, zähes Öl.

| | |
|---|---|
| Ausbeute: | 1.05 g (81 % der Theorie) |
| ¹H-NMR: | δ [CDCl₃] 0.90 (m, 6H), 1,62 (m, 4H), 3.37 (m, 4H), 3.72 (s, 3H), 4.72 (s, 2H), 7.39 (d, 1H), 7.59 (d, 1H). |

### Schritt 3: (5-(1,3-Dimethyl-pyrazolyl))-(2,4-Dibrom-3-(N,N-di-n-propylaminocarbonyl-methoxy)-benzoat

0.510 g 2,4-Dibrom-3-(N,N-di-n-propylaminocarbonyl-methoxy)-benzoesäure, 0.144 g 1,3-Dimethylpyrazolon, 0.228 g N'-(3-Dimethylaminopropyl)-N-ethylcarbodiimid Hydrochlorid und 0.001 g DMAP wurden in 15 ml CH₂Cl₂ 2 h bei RT gerührt. Anschliessend wurde mit 50 ml Wasser verdünnt und 30 min stark gerührt. Nach Ansäuern mit 0.5 N HCl wurden die Phasen getrennt. Die wäßrige Phase wurde mit CH₂Cl₂ extrahiert. Nach Trocknen der organischen Phasen über Na₂SO₄ und Einengen wurde (5-(1,3-Dimethyl-pyrazolyl))-(2,4-Dibrom-3-(N,N-di-n-propylaminocarbonyl-methoxy)-benzoat als gelbes Harz erhalten, das für die Folgeumsetzung ausreichend rein war.

| | |
|---|---|
| Ausbeute: | 0.440 g |
| ¹H-NMR: | δ [CDCl₃] 0.91 (m, 6H), 1,61 (m, 4H), 2.25 (s, 3H), 3.35 (m,4H), 3.72 (s, 3H), 4.69 (s, 2H), 6.08 (s, 1H), 7.60 (d, 1H), 7.68 (d, 1H). |

### Schritt 4: (1,3-Dimethyl-4-(2,4-Dibrom-3-(N,N-di-n-propylaminocarbonyl-methoxy)-benzoyl)-pyrazolon

0.440 g (5-(1,3-Dimethyl-pyrazolyl))-(2,4-Dibrom-3-(N,N-di-n-propylaminocarbonyl-methoxy)-benzoat wurden in 10 ml Acetonitril gelöst. Es wurden 3 Tropfen Acetoncyanhydrin sowie 0.142 g NEt₃ zugegeben. Die Mischung wurde 1 h bei RT gerührt, woraufhin 0.017 g KCN zugegeben wurden. Nach weiteren 3 h bei RT wurde vollständig eingeengt, der Rückstand in Wasser aufgenommen und mit 0.5 N HCl angesäuert. Anschliessend wurde mit CH₂Cl₂ extrahiert. Nach Trocknen der organischen Phasen über Na₂SO₄, Einengen und Chromatographie an reversed-phase-Kieselgel (Laufmittel: Acetonitril/Wasser-Gradient) erhielt man (1,3-Dimethyl-4-(2,4-Dibrom-3-(N,N-di-n-propylaminocarbonyl-methoxy)-benzoyl)-pyrazolon als farbloses, zähes Öl.

| | | |
|---|---|---|
| Ausbeute: | 0.205 g (ca. 44% der Theorie) | R^{f} (Essigester): 0.03 |
| ¹H-NMR: | δ [CDCl₃] 0.92 (m, 6H), 1,63 (m, 4H), 2.16 (s, 3H), 3.32 (m,4H), 3.64 (s, 3H), 4.71 (s, 2H), 6.94 (d, 1H), 7.65 (d, 1H). | |

Die in nachfolgenden Tabellen aufgeführten Beispiele wurden analog oben genannten Methoden hergestellt beziehungsweise sind analog den oben genannten Methoden erhältlich.

Die hier verwendeten Abkürzungen bedeuten:

| | | | |
|---|---|---|---|
| c = cyclo | i = iso | Bu = Butyl | Bz = Benzyl |
| Et = Ethyl | Me = Methyl | Ph = Phenyl | Pr = Propyl |
| EE = Essigester | Fp. = Festpunkt | | |

**Tabelle 1: Erfindungsgemäße Verbindungen der allgemeinen Formel (I), worin die Substituenten und Symbole folgende Bedeutungen haben:**

| | | | | | |
|---|---|---|---|---|---|
| R^{1c} = H | | | R⁴ = H | | R⁵ = Et |
| R⁶ = H | | | Y = O | | |
| 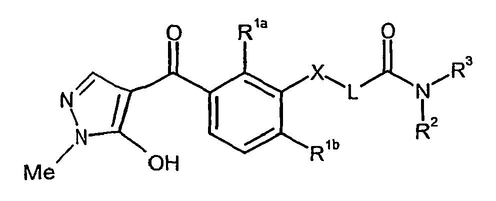 | | | | | |

| **Nr.** | **R^{1a}** | **R^{1b}** | **X-L** | **NR²R³** | **Physikal. Daten** |
|---|---|---|---|---|---|
| 1.1 | Cl | Cl | OCH₂ | NH₂ | |
| 1.2 | Br | Br | OCH₂ | NH₂ | |
| 1.3 | Me | Br | OCH₂ | NH₂ | |
| 1.4 | Cl | SO₂Me | OCH₂ | NH₂ | |
| 1.5 | Cl | SO₂Et | OCH₂ | NH₂ | |
| 1.6 | Me | SO₂Me | OCH₂ | NH₂ | |
| 1.6a | Me | Cl | OCH₂ | NHMe | |
| 1.6b | Me | Br | OCH₂ | NHMe | R^{f} (EE): 0,07 |
| 1.7 | Cl | Cl | OCH₂ | NHEt | |
| 1.8 | Cl | SO₂Me | OCH₂ | NHEt | |
| 1.8a | Me | Cl | OCH₂ | NHEt | |
| 1.8b | Me | Br | OCH₂ | NHEt | R^{f} (EE): 0,36 |
| 1.9 | Me | SO₂Me | OCH₂ | NHEt | |
| 1.9a | Me | Cl | OCH₂ | NH(Allyl) | |
| 1.9b | Me | Br | OCH₂ | NH(Allyl) | R^{f} (EE): 0,11 |
| 1.10 | Br | Br | OCH₂ | NH(i-Pr) | |
| 1.11 | Me | Br | OCH₂ | NH(i-Pr) | |
| 1.12 | Me | NO₂ | OCH₂ | NH(i-Pr) | |
| 1.13 | Cl | SO₂Et | OCH₂ | NH(i-Pr) | |
| 1.14 | Cl | Cl | OCH₂ | NH(c-Pr) | |
| 1.15 | Cl | Br | OCH₂ | NH(c-Pr) | |
| 1.15a | Me | Cl | OCH₂ | NH(c-Pr) | |
| 1.16 | Me | Br | OCH₂ | NH(c-Pr) | R^{f} (EE): 0,15 |
| 1.17 | Me | NO₂ | OCH₂ | NH(c-Pr) | |
| 1.18 | Cl | SO₂Me | OCH₂ | NH(c-Pr) | |
| 1.19 | Cl | Cl | OCH₂ | NMe₂ | R^{f} (EE): 0,02 |
| 1.20 | Br | Br | OCH₂ | NMe₂ | R^{f} (EE): 0,01 |
| 1.20a | Me | Cl | OCH₂ | NMe₂ | R^{f} (EE): 0,02 |
| 1.21 | Cl | Br | OCH₂ | NMe₂ | |
| 1.22 | Me | Br | OCH₂ | NMe₂ | R^{f} (EE): 0,01 |
| 1.23 | Cl | SO₂Me | OCH₂ | NMe₂ | |
| 1.24 | Me | SO₂Me | OCH₂ | NMe₂ | |
| 1.25 | Cl | SO₂Et | OCH₂ | NMe₂ | |
| 1.26 | Cl | Cl | OCH₂ | NEt₂ | |
| 1.27 | Br | Br | OCH₂ | NEt₂ | R^{f} (EE): 0,01 |
| 1.27a | Me | Cl | OCH₂ | NEt₂ | R^{f} (EE): 0,02 |
| 1.28 | Cl | Br | OCH₂ | NEt₂ | |
| 1.29 | Me | Br | OCH₂ | NEt₂ | R^{f} (EE): 0,03 |
| 1.30 | Cl | SO₂Me | OCH₂ | NEt₂ | |
| 1.31 | Me | SO₂Me | OCH₂ | NEt₂ | |
| 1.32 | Cl | SO₂Et | OCH₂ | NEt₂ | |
| 1.33 | Cl | Cl | OCH₂ | N(n-Pr)₂ | R^{f} (EE): 0,02 |
| 1.34 | Br | Br | OCH₂ | N(n-Pr)₂ | R^{f} (EE): 0,03 |
| 1.35 | Cl | Br | OCH₂ | N(n-Pr)₂ | |
| 1.36 | Me | Br | OCH₂ | N(n-Pr)₂ | |
| 1.37 | Cl | SO₂Me | OCH₂ | N(n-Pr)₂ | |
| 1.38 | Me | SO₂Me | OCH₂ | N(n-Pr)₂ | |
| 1.39 | Cl | SO₂Et | OCH₂ | N(n-Pr)₂ | R^{f} (EE): 0,06 |
| 1.40 | Cl | Cl | OCH₂ | N(i-Pr)₂ | R^{f} (EE): 0,03 |
| 1.41 | Me | Br | OCH₂ | N(i-Pr)₂ | |
| 1.42 | Me | NO₂ | OCH₂ | N(i-Pr)₂ | |
| 1.43 | NO₂ | Cl | OCH₂ | N(i-Pr)₂ | |
| 1.44 | NO₂ | Br | OCH₂ | N(i-Pr)₂ | |
| 1.45 | Me | SO₂Me | OCH₂ | N(i-Pr)₂ | |
| 1.46 | Cl | SO₂Et | OCH₂ | N(i-Pr)₂ | R^{f} (EE): 0,02 |
| 1.47 | Cl | Cl | OCH₂ | NMePh | R^{f} (EE): 0,07 |
| 1.48 | Me | Br | OCH₂ | NMePh | |
| 1.49 | Me | NO₂ | OCH₂ | NMePh | |
| 1.50 | NO₂ | Cl | OCH₂ | NMePh | |
| 1.51 | NO₂ | Br | OCH₂ | NMePh | |
| 1.52 | Me | SO₂Me | OCH₂ | NMePh | |
| 1.53 | Cl | SO₂Et | OCH₂ | NMePh | R^{f} (EE): 0,01 |
| 1.54 | Cl | Cl | OCH₂ | 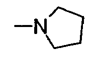 | R^{f} (EE): 0,02 |
| 1.55 | Br | Br | OCH₂ | 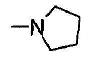 | |
| 1.56 | Cl | Br | OCH₂ | 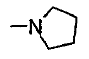 | |
| 1.57 | Me | Br | OCH₂ | 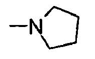 | |
| 1.58 | Cl | SO₂Me | OCH₂ | 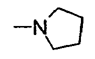 | R^{f} (EE): 0,02 |
| 1.59 | Me | SO₂Me | OCH₂ | 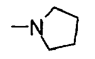 | |
| 1.60 | Cl | SO₂Et | OCH₂ | 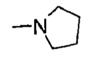 | R^{f} (EE): 0,01 |
| 1.61 | Cl | Cl | OCH₂ | 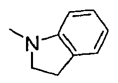 | R^{f} (EE): 0,04 |
| 1.62 | Br | Br | OCH₂ | 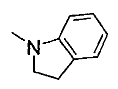 | |
| 1.63 | Cl | Br | OCH₂ | 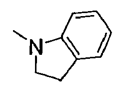 | |
| 1.64 | Me | Br | OCH₂ | 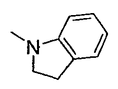 | |
| 1.65 | Cl | SO₂Me | OCH₂ | 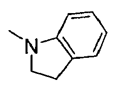 | R^{f} (EE): 0,04 |
| 1.66 | Me | SO₂Me | OCH₂ | 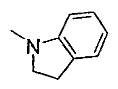 | |
| 1.67 | Cl | SO₂Et | OCH₂ | 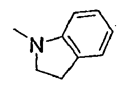 | |
| 1.68 | Cl | Cl | OCH₂ | 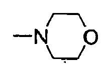 | |
| 1.69 | Me | Br | OCH₂ | 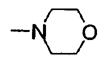 | |
| 1.70 | Me | NO₂ | OCH₂ | 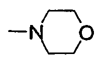 | |
| 1.71 | NO₂ | Cl | OCH₂ | 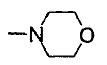 | |
| 1.72 | NO₂ | Br | OCH₂ | 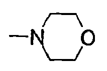 | |
| 1.73 | Me | SO₂M | OCH₂ | 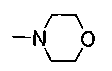 | |
| 1.74 | Cl | SO₂Et | OCH₂ | 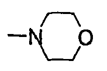 | |
| 1.75 | Cl | Cl | OC(Me)H | NH₂ | |
| 1.76 | Br | Br | OCH(Me) | NH₂ | |
| 1.77 | Me | Br | OCH(Me) | NH₂ | |
| 1.78 | Cl | SO₂Me | OCH(Me) | NH₂ | |
| 1.79 | Cl | SO₂Et | OCH(Me) | NH₂ | |
| 1.80 | Me | SO₂Me | QCH(Me) | NH₂ | |
| 1.80a | Cl | Cl | OCH(Me) | NHMe | |
| 1.80b | Cl | SO₂Me | OCH(Me) | NHMe | |
| 1.80c | Me | Cl | OCH(Me) | NHMe | |
| 1.80d | Me | Br | OCH(Me) | NHMe | |
| 1.80e | Me | SO₂Me | OCH(Me) | NHMe | |
| 1.81 | Cl | Cl | OCH(Me) | NHEt | |
| 1.82 | Cl | SO₂Me | OCH(Me) | NHEt | |
| 1.82a | Me | Cl | OCH(Me) | NHEt | |
| 1.82b | Me | Br | OCH(Me) | NHEt | R^{f} (EE): 0,05 |
| 1.82c | Cl | Cl | OCH(Me) | NH(Allyl) | R^{f} (EE): 0,05 |
| 1.82d | Cl | SO₂Me | OCH(Me) | NH(Allyl) | |
| 1.82e | Me | Cl | OCH(Me) | NH(Allyl) | |
| 1.82f | Me | Br | OCH(Me) | NH(Allyl) | R^{f} (EE): 0,24 |
| 1.82g | Me | SO₂Me | OCH(Me) | NH(Allyl) | |
| 1.83 | Me | SO₂Me | OCH(Me) | NHEt | |
| 1.84 | Br | Br | OCH(Me) | NH(i-Pr) | |
| 1.85 | Me | Br | OCH(Me) | NH(i-Pr) | |
| 1.86 | Me | NO₂ | OCH(Me) | NH(i-Pr) | |
| 1.87 | Cl | SO₂Et | OCH(Me) | NH(i-Pr) | |
| 1.88 | Cl | Cl | OCH(Me) | NH(c-Pr) | R^{f} (EE): 0,03 |
| 1.89 | Cl | Br | OCH(Me) | NH(c-Pr) | |
| 1.90 | Me | Br | OCH(Me) | NH(c-Pr) | |
| 1.91 | Me | NO₂ | OCH(Me) | NH(c-Pr) | |
| 1.92 | Cl | SO₂M | OCH(Me) | NH(c-Pr) | |
| 1.93 | Cl | Cl | OCH(Me) | NMe₂ | |
| 1.94 | Br | Br | OCH(Me) | NMe₂ | |
| 1.95 | Cl | Br | OCH(Me) | NMe₂ | |
| 1.96 | Me | Br | OCH(Me) | NMe₂ | R^{f} (EE): 0,03 |
| 1.97 | Cl | SO₂M | OCH(Me) | NMe₂ | |
| 1.98 | Me | SO₂Me | OCH(Me) | NMe₂ | |
| 1.99 | Cl | SO₂Et | OCH(Me) | NMe₂ | |
| 1.100 | Cl | Cl | OCH(Me) | NEt₂ | |
| 1.101 | Br | Br | OCH(Me) | NEt₂ | |
| 1.102 | Cl | Br | OCH(Me) | NEt₂ | |
| 1.103 | Me | Br | OCH(Me) | NEt₂ | |
| 1.104 | Cl | SO₂Me | OCH(Me) | NEt₂ | |
| 1.105 | Me | SO₂Me | OCH(Me) | NEt₂ | |
| 1.106 | Cl | SO₂Et | OCH(Me) | NEt₂ | |
| 1.107 | Cl | Cl | OCH(Me) | N(n-Pr)₂ | |
| 1.108 | Br | Br | OCH(Me) | N(n-Pr)₂ | |
| 1.109 | Cl | Br | OCH(Me) | N(n-Pr)₂ | |
| 1.110 | Me | Br | OCH(Me) | N(n-Pr)₂ | |
| 1.111 | Cl | SO₂Me | OCH(Me) | N(n-Pr)₂ | |
| 1.112 | Me | SO₂Me | OCH(Me) | N(n-Pr)₂ | |
| 1.113 | Cl | SO₂Et | OCH(Me) | N(n-Pr)₂ | |
| 1.114 | Cl | Cl | OCH(Me) | N(i-Pr)₂ | |
| 1.115 | Me | Br | OCH(Me) | N(i-Pr)₂ | |
| 1.116 | Me | NO₂ | OCH(Me) | N(i-Pr)₂ | |
| 1.117 | NO₂ | Cl | OCH(Me) | N(i-Pr)₂ | |
| 1.118 | NO₂ | Br | OCH(Me) | N(i-Pr)₂ | |
| 1.119 | Me | SO₂Me | OCH(Me) | N(i-Pr)₂ | |
| 1.120 | Cl | SO₂Et | OCH(Me) | N(i-Pr)₂ | |
| 1.121 | Cl | Cl | OCH(Me) | NMePh | |
| 1.122 | Me | Br | OCH(Me) | NMePh | |
| 1.123 | Me | NO₂ | OCH(Me) | NMePh | |
| 1.124 | NO₂ | Cl | OCH(Me) | NMePh | |
| 1.125 | NO₂ | Br | OCH(Me) | NMePh | |
| 1.126 | Me | SO₂Me | OCH(Me) | NMePh | |
| 1.127 | Cl | SO₂Et | OCH(Me) | NMePh | |
| 1.128 | Cl | Cl | OCH(Me) | 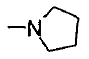 | |
| 1.129 | Br | Br | OCH(Me) | 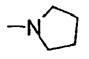 | |
| 1.130 | Cl | Br | OCH(Me) | 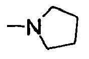 | |
| 1.131 | Me | Br | OCH(Me) | 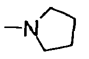 | |
| 1.132 | Cl | SO₂Me | OCH(Me) | 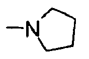 | |
| 1.133 | Me | SO₂Me | OCH(Me) | 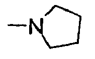 | |
| 1.134 | Cl | SO₂Et | OCH(Me) | 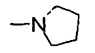 | |
| 1.135 | Cl | Cl | OCH(Me) | 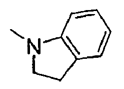 | |
| 1.136 | Br | Br | OCH(Me) | 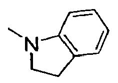 | |
| 1.137 | Cl | Br | OCH(Me) | 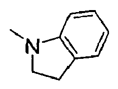 | |
| 1.138 | Me | Br | OCH(Me) | 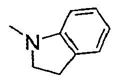 | |
| 1.139 | Cl | SO₂Me | OCH(Me) | 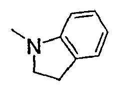 | |
| 1.140 | Me | SO₂Me | OCH(Me) | 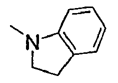 | |
| 1.141 | Cl | SO₂Et | OCH(Me) | 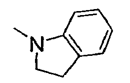 | |
| 1.142 | Cl | Cl | OCH(Me) | 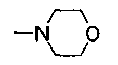 | |
| 1.143 | Me | Br | OCH(Me) | 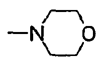 | |
| 1.144 | Me | NO₂ | OCH(Me) | 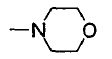 | |
| 1.145 | NO₂ | Cl | OCH(Me) | 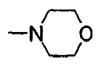 | |
| 1.146 | NO₂ | Br | OCH(Me) | 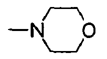 | |
| 1.147 | Me | SO₂Me | OCH(Me) | 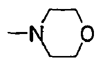 | |
| 1.148 | Cl | SO₂E | OCH(Me) | 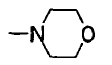 | |
| 1.149 | Cl | Cl | OCH₂CH₂ | NH₂ | |
| 1.150 | Br | Br | OCH₂CH₂ | NH₂ | |
| 1.151 | Me | Br | OCH₂CH₂ | NH₂ | |
| 1.152 | Cl | SO₂Me | OCH₂CH₂ | NH₂ | |
| 1.153 | Cl | SO₂Et | OCH₂CH₂ | NH₂ | |
| 1.154 | Me | SO₂Me | OCH₂CH₂ | NH₂ | |
| 1.155 | Cl | Cl | OCH₂CH₂ | NHEt | |
| 1.156 | Cl | SO₂Me | OCH₂CH₂ | NHEt | |
| 1.157 | Me | SO₂Me | OCH₂CH₂ | NHEt | |
| 1.158 | Br | Br | OCH₂CH₂ | NH(i-Pr) | |
| 1.159 | Me | Br | OCH₂CH₂ | NH(i-Pr) | |
| 1.160 | Me | NO₂ | OCH₂CH₂ | NH(i-Pr) | |
| 1.161 | Cl | SO₂Et | OCH₂CH₂ | NH(i-Pr) | |
| 1.162 | Cl | Cl | OCH₂CH₂ | NH(c-Pr) | |
| 1.163 | Cl | Br | OCH₂CH₂ | NH(c-Pr) | |
| 1.164 | Me | Br | OCH₂CH₂ | NH(c-Pr) | |
| 1.165 | Me | NO₂ | OCH₂CH₂ | NH(c-Pr) | |
| 1.166 | Cl | SO₂Me | OCH₂CH₂ | NH(c-Pr) | |
| 1.167 | Cl | Cl | OCH₂CH₂ | NMe₂ | |
| 1.168 | Br | Br | OCH₂CH₂ | NMe₂ | |
| 1.169 | Cl | Br | OCH₂CH₂ | NMe₂ | |
| 1.170 | Me | Br | OCH₂CH₂ | NMe₂ | |
| 1.171 | Cl | SO₂Me | OCH₂CH₂ | NMe₂ | |
| 1.172 | Me | SO₂Me | OCH₂CH₂ | NMe₂ | |
| 1.173 | Cl | SO₂Et | OCH₂CH₂ | NMe₂ | |
| 1.174 | Cl | Cl | OCH₂CH₂ | NEt₂ | |
| 1.175 | Br | Br | OCH₂CH₂ | NEt₂ | |
| 1.176 | Cl | Br | OCH₂CH₂ | NEt₂ | |
| 1.177 | Me | Br | OCH₂CH₂ | NEt₂ | |
| 1.178 | Cl | SO₂Me | OCH₂CH₂ | NEt₂ | |
| 1.179 | Me | SO₂Me | OCH₂CH₂ | NEt₂ | |
| 1.180 | Cl | SO₂Et | OCH₂CH₂ | NEt₂ | |
| 1.181 | Cl | Cl | OCH₂CH₂ | N(n-Pr)₂ | |
| 1.182 | Br | Br | OCH₂CH₂ | N(n-Pr)₂ | |
| 1.183 | Cl | Br | OCH₂CH₂ | N(n-Pr)₂ | |
| 1.184 | Me | Br | OCH₂CH₂ | N(n-Pr)₂ | |
| 1.185 | Cl | SO₂Me | OCH₂CH₂ | N(n-Pr)₂ | |
| 1.186 | Me | SO₂Me | OCH₂CH₂ | N(n-Pr)₂ | |
| 1.187 | Cl | SO₂Et | OCH₂CH₂ | N(n-Pr)₂ | |
| 1.188 | Cl | Cl | OCH₂CH₂ | N(i-Pr)₂ | |
| 1.189 | Me | Br | OCH₂CH₂ | N(i-Pr)₂ | |
| 1.190 | Me | NO₂ | OCH₂CH₂ | N(i-Pr)₂ | |
| 1.191 | NO₂ | Cl | OCH₂CH₂ | N(i-Pr)₂ | |
| 1.192 | NO₂ | Br | OCH₂CH₂ | N(i-Pr)₂ | |
| 1.193 | Me | SO₂Me | OCH₂CH₂ | N(i-Pr)₂ | |
| 1.194 | Cl | SO₂Et | OCH₂CH₂ | N(i-Pr)₂ | |
| 1.195 | Cl | Cl | OCH₂CH₂ | NMePh | |
| 1.196 | Me | Br | OCH₂CH₂ | NMePh | |
| 1.197 | Me | NO₂ | OCH₂CH₂ | NMePh | |
| 1.198 | NO₂ | Cl | OCH₂CH₂ | NMePh | |
| 1.199 | NO₂ | Br | OCH₂CH₂ | NMePh | |
| 1.200 | Me | SO₂Me | OCH₂CH₂ | NMePh | |
| 1.201 | Cl | SO₂Et | OCH₂CH₂ | NMePh | |
| 1.202 | Cl | Cl | OCH₂CH₂ | 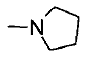 | |
| 1.203 | Br | Br | OCH₂CH₂ | 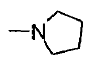 | |
| 1.204 | Cl | Br | OCH₂CH₂ | 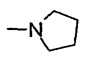 | |
| 1.205 | Me | Br | OCH₂CH₂ | 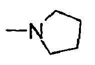 | |
| 1.206 | Cl | SO₂Me | OCH₂CH₂ | 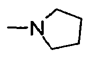 | |
| 1.207 | Me | SO₂Me | OCH₂CH₂ | 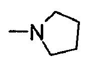 | |
| 1.208 | Cl | SO₂Et | OCH₂CH₂ | 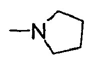 | |
| 1.209 | Cl | Cl | OCH₂CH₂ | 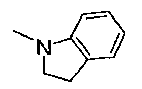 | |
| 1.210 | Cl | Cl | OCH₂CH=CH | NMe₂ | |
| 1.211 | Cl | SO₂Me | OCH₂CH=CH | NMe₂ | |
| 1.212 | Me | Cl | OCH₂CH=CH | NMe₂ | |
| 1.213 | Me | Br | OCH₂CH=CH | NMe₂ | |
| 1.214 | Me | SO₂Me | OCH₂CH=CH | NMe₂ | |
| 1.215 | Cl | Cl | OCH₂CH=CH | NEt₂ | |
| 1.216 | Cl | SO₂Me | OCH₂CH=CH | NEt₂ | |
| 1.217 | Me | Cl | OCH₂CH=CH | NEt₂ | |
| 1.218 | Me | Br | OCH₂CH=CH | NEt₂ | |
| 1.219 | Me | SO₂Me | OCH₂CH=CH | NEt₂ | |
| 1.220 | Cl | Cl | OCH₂CH=CH | Nh(c-Pr) | |
| 1.221 | Cl | SO₂Me | OCH₂CH=CH | Nh(c-Pr) | |
| 1.222 | Me | Cl | OCH₂CH=CH | Nh(c-Pr) | |
| 1.223 | Me | Br | OCH₂CH=CH | Nh(c-Pr) | |
| 1.224 | Me | SO₂Me | OCH₂CH=CH | Nh(c-Pr) | |

**Tabelle 2: Erfindungsgemäße Verbindungen der Formel (I), worin die Substituenten und Symbole folgende Bedeutungen haben:**

| | | | | | |
|---|---|---|---|---|---|
| R^{1c} = H | | | R⁴ = H | | R⁵ = Et |
| R⁶ = H | | | Y = O | | |
| 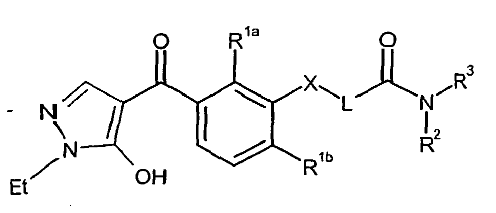 | | | | | |

| **Nr.** | **R^{1a}** | **R^{1b}** | **X-L** | **NR²R³** | **Physikal. Daten** |
|---|---|---|---|---|---|
| 2.1 | Cl | Cl | OCH₂ | NH₂ | |
| 2.2 | Br | Br | OCH₂ | NH₂ | |
| 2.3 | Me | Br | OCH₂ | NH₂ | |
| 2.4 | Cl | SO₂Me | OCH₂ | NH₂ | |
| 2.5 | Cl | SO₂Et | OCH₂ | NH₂ | |
| 2.6 | Me | SO₂Me | OCH₂ | NH₂ | |
| 2.6a | Me | Cl | OCH₂ | NHMe | |
| 2.6b | Me | Br | OCH₂ | NHMe | |
| 2.7 | Cl | Cl | OCH₂ | NHEt | |
| 2.8 | Cl | SO₂Me | OCH₂ | NHEt | |
| 2.8a | Me | Cl | OCH₂ | NHEt | |
| 2.8b | Me | Br | OCH₂ | NHEt | |
| 2.9 | Me | SO₂Me | OCH₂ | NHEt | |
| 2.9a | Me | Cl | OCH₂ | NH(Allyl) | |
| 2.9b | Me | Br | OCH₂ | NH(Allyl) | |
| 2.10 | Br | Br | OCH₂ | NH(i-Pr) | |
| 2.11 | Me | Br | OCH₂ | NH(i-Pr) | |
| 2.12 | Me | NO₂ | OCH₂ | NH(i-Pr) | |
| 2.13 | Cl | SO₂Et | OCH₂ | NH(i-Pr) | |
| 2.14 | Cl | Cl | OCH₂ | NH(c-Pr) | |
| 2.15 | Cl | Br | OCH₂ | NH(c-Pr) | |
| 2.15a | Me | Cl | OCH₂ | NH(c-Pr) | |
| 2.16 | Me | Br | OCH₂ | NH(c-Pr) | |
| 2.17 | Me | NO₂ | OCH₂ | NH(c-Pr) | |
| 2.18 | Cl | SO₂Me | OCH₂ | NH(c-Pr) | |
| 2.19 | Cl | Cl | OCH₂ | NMe₂ | R^{f} (EE): 0,27 |
| 2.20 | Br | Br | OCH₂ | NMe₂ | |
| 2.20a | Me | Cl | OCH₂ | NMe₂ | R^{f} (EE): 0,01 |
| 2.21 | Cl | Br | OCH₂ | NMe₂ | |
| 2.22 | Me | Br | OCH₂ | NMe₂ | |
| 2.23 | Cl | SO₂Me | OCH₂ | NMe₂ | |
| 2.24 | Me | SO₂Me | OCH₂ | NMe₂ | |
| 2.25 | Cl | SO₂Et | OCH₂ | NMe₂ | |
| 2.26 | Cl | Cl | OCH₂ | NEt₂ | |
| 2.27 | Br | Br | OCH₂ | NEt₂ | |
| 2.27a | Me | Cl | OCH₂ | NEt₂ | R^{f} (EE): 0,01 |
| 2.28 | Cl | Br | OCH₂ | NEt₂ | |
| 2.29 | Me | Br | OCH₂ | NEt₂ | |
| 2.30 | Cl | SO₂Me | OCH₂ | NEt₂ | R^{f} (EE): 0,04 |
| 2.31 | Me | SO₂Me | OCH₂ | NEt₂ | |
| 2.32 | Cl | SO₂Et | OCH₂ | NEt₂ | |
| 2.33 | Cl | Cl | OCH₂ | N(n-Pr)₂ | |
| 2.34 | Br | Br | OCH₂ | N(n-Pr)₂ | |
| 2.35 | Cl | Br | OCH₂ | N(n-Pr)₂ | |
| 2.36 | Me | Br | OCH₂ | N(n-Pr)₂ | |
| 2.37 | Cl | SO₂Me | OCH₂ | N(n-Pr)₂ | |
| 2.38 | Me | SO₂Me | OCH₂ | N(n-Pr)₂ | |
| 2.39 | Cl | SO₂Et | OCH₂ | N(n-Pr)₂ | |
| 2.40 | Cl | Cl | OCH₂ | N(i-Pr)₂ | |
| 2.41 | Me | Br | OCH₂ | N(i-Pr)₂ | |
| 2.42 | Me | NO₂ | OCH₂ | N(i-Pr)₂ | |
| 2.43 | NO₂ | Cl | OCH₂ | N(i-Pr)₂ | |
| 2.44 | NO₂ | Br | OCH₂ | N(i-Pr)₂ | |
| 2.45 | Me | SO₂Me | OCH₂ | N(i-Pr)₂ | |
| 2.46 | Cl | SO₂Et | OCH₂ | N(i-Pr)₂ | |
| 2.47 | Cl | Cl | OCH₂ | NMePh | |
| 2.48 | Me | Br | OCH₂ | NMePh | |
| 2.49 | Me | NO₂ | OCH₂ | NMePh | |
| 2.50 | NO₂ | Cl | OCH₂ | NMePh | |
| 2.51 | NO₂ | Br | OCH₂ | NMePh | |
| 2.52 | Me | SO₂Me | OCH₂ | NMePh | |
| 2.53 | Cl | SO₂Et | OCH₂ | NMePh | |
| 2.54 | Cl | Cl | OCH₂ | 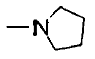 | |
| 2.55 | Br | Br | OCH₂ | 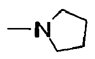 | |
| 2.56 | Cl | Br | OCH₂ | 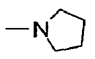 | |
| 2.57 | Me | Br | OCH₂ | 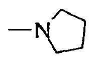 | |
| 2.58 | Cl | SO₂Me | OCH₂ | 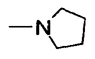 | |
| 2.59 | Me | SO₂Me | OCH₂ | 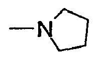 | |
| 2.60 | Cl | SO₂Et | OCH₂ | 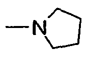 | |
| 2.61 | Cl | Cl | OCH₂ | 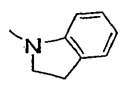 | |
| 2.62- | Br | Br | OCH₂ | 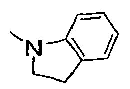 | |
| 2.63 | Cl | Br | OCH₂ | 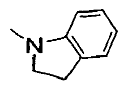 | |
| 2.64 | Me | Br | OCH₂ | 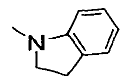 | |
| 2.65 | Cl | SO₂Me | OCH₂ | 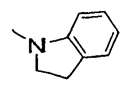 | |
| 2.66 | Me | SO₂Me | OCH₂ | 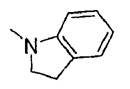 | |
| 2.67 | Cl | SO₂Et | OCH₂ | 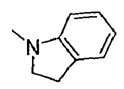 | |
| 2.68 | Cl | Cl | OCH₂ | 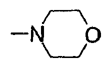 | |
| 2.69 | Me | Br | OCH₂ | 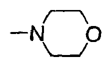 | |
| 2.70 | Me | NO₂ | OCH₂ | 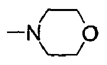 | |
| 2.71 | NO₂ | Cl | OCH₂ | 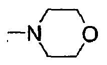 | |
| 2.72 | NO₂ | Br | OCH₂ | 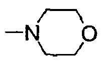 | |
| 2.73 | Me | SO₂Me | OCH₂ | 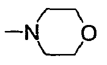 | |
| 2.74 | Cl | SO₂ET | OCH₂ | 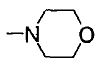 | |
| 2.75 | Cl | Cl | OCH(Me) | NH₂ | |
| 2.76 | Br | Br | OCH(Me) | NH₂ | |
| 2.77 | Me | Br | OCH(Me) | NH₂ | |
| 2.78 | Cl | SO₂Me | OCH(Me) | NH₂ | |
| 2.79 | Cl | SO₂Et | OCH(Me) | NH₂ | |
| 2.80 | Me | SO₂Me | OCH(Me) | NH₂ | |
| 2.80a | Cl | Cl | OCH(Me) | NHMe | R^{f} (EE): 0,26 |
| 2.80b | Cl | SO₂Me | OCH(Me) | NHMe | |
| 2.80c | Me | Cl | OCH(Me) | NHMe | |
| 2.80d | Me | Br | OCH(Me) | NHMe | |
| 2.80e | Me | SO₂Me | OCH(Me) | NHMe | |
| 2.81 | Cl | Cl | OCH(Me) | NHEt | R^{f} (EE): 0,01 |
| 2.82 | Cl | SO₂Me | OCH(Me) | NHEt | |
| 2.82a | Me | Cl | OCH(Me) | NHEt | |
| 2.82b | Me | Br | OCH(Me) | NHEt | |
| 2.82c | Cl | Cl | OCH(Me) | NH(Allyl) | |
| 2.82d | Cl | SO₂Me | OCH(Me) | NH(Allyl) | |
| 2.82e | Me | Cl | OCH(Me) | NH(Allyl) | |
| 2.82f | Me | Br | OCH(Me) | NH(Allyl) | |
| 2.82g | Me | SO₂Me | OCH(Me) | NH(Allyl) | |
| 2.83 | Me | SO₂Me | OCH(Me) | NHEt | |
| 2.84 | Br | Br | OCH(Me) | NH(i-Pr) | |
| 2.85 | Me | Br | OCH(Me) | NH(i-Pr) | |
| 2.86 | Me | NO₂ | OCH(Me) | NH(i-Pr) | |
| 2.87 | Cl | SO₂Et | OCH(Me) | NH(i-Pr) | |
| 2.88 | Cl | Cl | OCH(Me) | NH(c-Pr) | R^{f} (EE): 0,03 |
| 2.89 | Cl | Br | OCH(Me) | NH(c-Pr) | |
| 2.90 | Me | Br | OCH(Me) | NH(c-Pr) | |
| 2.91 | Me | NO₂ | OCH(Me) | NH(c-Pr) | |
| 2.92 | Cl | SO₂Me | OCH(Me) | NH(c-Pr) | |
| 2.93 | Cl | Cl | OCH(Me) | NMe₂ | R^{f} (EE): 0,28 |
| 2.94 | Br | Br | OCH(Me) | NMe₂ | |
| 2.95 | Cl | Br | OCH(Me) | NMe₂ | |
| 2.96 | Me | Br | OCH(Me) | NMe₂ | R^{f} (EE): 0,04 |
| 2.97 | Cl | SO₂Me | OCH(Me) | NMe₂ | |
| 2.98 | Me | SO₂Me | OCH(Me) | NMe₂ | |
| 2.99 | Cl | SO₂Et | OCH(Me) | NMe₂ | |
| 2.100 | Cl | Cl | OCH(Me) | NEt₂ | |
| 2.101 | Br | Br | OCH(Me) | NEt₂ | |
| 2.102 | Cl | Br | OCH(Me) | NEt₂ | |
| 2.103 | Me | Br | OCH(Me) | NEt₂ | |
| 2.104 | Cl | SO₂Me | OCH(Me) | NEt₂ | |
| 2.105 | Me | SO₂Me | OCH(Me) | NEt₂ | |
| 2.106 | Cl | SO₂Et | OCH(Me) | NEt₂ | |
| 2.107 | Cl | Cl | OCH(Me) | N(n-Pr)₂ | |
| 2.108 | Br | Br | OCH(Me) | N(n-Pr)₂ | |
| 2.109 | Cl | Br | OCH(Me) | N(n-Pr)₂ | |
| 2.110 | Me | Br | OCH(Me) | N(n-Pr)₂ | |
| 2.111 | Cl | SO₂Me | OCH(Me) | N(n-Pr)₂ | |
| 2.112 | Me | SO₂Me | OCH(Me) | N(n-Pr)₂ | |
| 2.113 | Cl | SO₂Et | OCH(Me) | N(n-Pr)₂ | |
| 2.114 | Cl | Cl | OCH(Me) | N(i-Pr)₂ | |
| 2.115 | Me | Br | OCN(Me) | N(i-Pr)₂ | |
| 2.116 | Me | NO₂ | OCH(Me) | N(i-Pr)₂ | |
| 2.117 | NO₂ | Cl | OCH(Me) | N(i-Pr)₂ | |
| 2.118 | NO₂ | Br | OCH(Me) | N(i-Pr)₂ | |
| 2.119 | Me | SO₂Me | OCH(Me) | N(i-Pr)₂ | |
| 2.120 | Cl | SO₂E | OCH(Me) | N(i-Pr)₂ | |
| 2.121 | Cl | Cl | OCH(Me) | NMePh | |
| 2.122 | Me | Br | OCH(Me) | NMePh | |
| 2.123 | Me | NO₂ | OCH(Me) | NMePh | |
| 2.124 | NO₂ | Cl | OCH(Me) | NMePh | |
| 2.125 | NO₂ | Br | OCH(Me) | NMePh | |
| 2.126 | Me | SO₂Me | OCH(Me) | NMePh | |
| 2.127 | Cl | SO₂E | OCH(Me) | NMePh | |
| 2.128 | Cl | Cl | OCH(Me) | 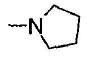 | |
| 2.129 | Br | Br | OCH(Me) | 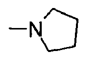 | |
| 2.130 | Cl | Br | OCH(Me) | 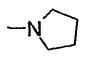 | |
| 2.131 | Me | Br | OCH(Me) | 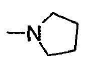 | |
| 2.132 | Cl | SO₂M | OCH(Me) | 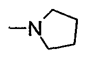 | |
| 2.133 | Me | SO₂Me | OCH(Me) | 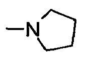 | |
| 2.134 | Cl | SO₂Et | OCH(Me) | 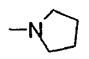 | |
| 2.135 | Cl | Cl | OCH(Me) | 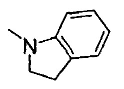 | |
| 2.136 | Br | Br | OCH(Me) | 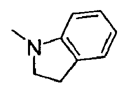 | |
| 2.137 | Cl | Br | OCH(Me) | 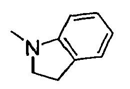 | |
| 2.138 | Me | Br | OCH(Me) | 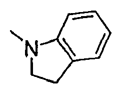 | |
| 2.139 | Cl | SO₂Me | OCH(Me) | 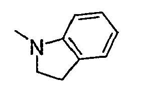 | |
| 2.140 | Me | SO₂Me | OCH(Me) | 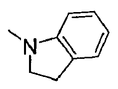 | |
| 2.141 | Cl | SO₂ET | OCH(Me) | 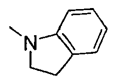 | |
| 2.142 | Cl | Cl | OCH(Me) | 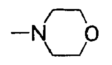 | |
| 2.143 | Me | Br | OCH(Me) | 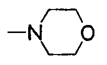 | |
| 2.144 | Me | NO₂ | OCH(Me) | 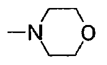 | |
| 2.145 | NO₂ | Cl | OCH(Me) | 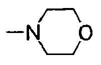 | |
| 2.146 | NO₂ | Br | OCH(Me) | 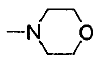 | |
| 2.147 | Me | SO₂Me | OCH(Me) | 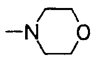 | |
| 2.148 | Cl | SO₂Et | OCH(Me) | 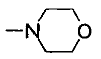 | |
| 2.149 | Cl | Cl | OCH₂CH₂ | NH₂ | |
| 2.150 | Br | Br | OCH₂CH₂ | NH₂ | |
| 2.151 | Me | Br | OCH₂CH₂ | NH₂ | |
| 2.152 | Cl | SO₂Me | OCH₂CH₂ | NH₂ | |
| 2.153 | Cl | SO₂Et | OCH₂CH₂ | NH₂ | |
| 2.154 | Me | SO₂Me | OCH₂CH₂ | NH₂ | |
| 2.155 | Cl | Cl | OCH₂CH₂ | NHEt | |
| 2.156 | Cl | SO₂Me | OCH₂CH₂ | NHEt | |
| 2.157 | Me | SO₂Me | OCH₂CH₂ | NHEt | |
| 2.158 | Br | Br | OCH₂CH₂ | NH(i-Pr) | |
| 2.159 | Me | Br | OCH₂CH₂ | NH(i-Pr) | |
| 2.160 | Me | NO₂ | OCH₂CH₂ | NH(i-Pr) | |
| 2.161 | Cl | SO₂Et | OCH₂CH₂ | NH(i-Pr) | |
| 2.162 | Cl | Cl | OCH₂CH₂ | NH(c-Pr) | |
| 2.163 | Cl | Br | OCH₂CH₂ | NH(c-Pr) | |
| 2.164 | Me | Br | OCH₂CH₂ | NH(c-Pr) | |
| 2.165 | Me | NO₂ | OCH₂CH₂ | NH(c-Pr) | |
| 2.166 | Cl | SO₂Me | OCH₂CH₂ | NH(c-Pr) | |
| 2.167 | Cl | Cl | OCH₂CH₂ | NMe₂ | |
| 2.168 | Br | Br | OCH₂CH₂ | NMe₂ | |
| 2.169 | Cl | Br | OCH₂CH₂ | NMe₂ | |
| 2.170 | Me | Br | OCH₂CH₂ | NMe₂ | |
| 2.171 | Cl | SO₂Me | OCH₂CH₂ | NMe₂ | |
| 2.172 | Me | SO₂Me | OCH₂CH₂ | NMe₂ | |
| 2.173 | Cl | SO₂Et | OCH₂CH₂ | NMe₂ | |
| 2.174 | Cl | Cl | OCH₂CH₂ | NEt₂ | |
| 2.175 | Br | Br | OCH₂CH₂ | NEt₂ | |
| 2.176 | Cl | Br | OCH₂CH₂ | NEt₂ | |
| 2.177 | Me | Br | OCH₂CH₂ | NEt₂ | |
| 2.178 | Cl | SO₂Me | OCH₂CH₂ | NEt₂ | |
| 2.179 | Me | SO₂Me | OCH₂CH₂ | NEt₂ | |
| 2.180 | Cl | SO₂Et | OCH₂CH₂ | NEt₂ | |
| 2.181 | Cl | Cl | OCH₂CH₂ | N(n-Pr)₂ | |
| 2.182 | Br | Br | OCH₂CH₂ | N(n-Pr)₂ | |
| 2.183 | Cl | Br | OCH₂CH₂ | N(n-Pr)₂ | |
| 2.184 | Me | Br | OCH₂CH₂ | N(n-Pr)₂ | |
| 2.185 | Cl | SO₂Me | OCH₂CH₂ | N(n-Pr)₂ | |
| 2.186 | Me | SO₂Me | OCH₂CH₂ | N(n-Pr)₂ | |
| 2.187 | Cl | SO₂Et | OCH₂CH₂ | N(n-Pr)₂ | |
| 2.188 | Cl | Cl | OCH₂CH₂ | N(i-Pr)₂ | |
| 2.189 | Me | Br | OCH₂CH₂- | N(i-Pr)₂ | |
| 2.190 | Me | NO₂ | OCH₂CH₂ | N(i-Pr)₂ | |
| 2.191 | NO₂ | Cl | OCH₂CH₂ | N(i-Pr)₂ | |
| 2.192 | NO₂ | Br | OCH₂CH₂ | N(i-Pr)₂ | |
| 2.193 | Me | SO₂Me | OCH₂CH₂ | N(i-Pr)₂ | |
| 2.194 | Cl | SO₂Et. | OCH₂CH₂ | N(i-Pr)₂ | |
| 2.195 | Cl | Cl | OCH₂CH₂ | NMePh | |
| 2.196 | Me | Br | OCH₂CH₂ | NMePh | |
| 2.197 | Me | NO₂ | OCH₂CH₂ | NMePh | |
| 2.198 | NO₂ | Cl | OCH₂CH₂ | NMePh | |
| 2.199 | NO₂ | Br | OCH₂CH₂ | NMePh | |
| 2.200 | Me | SO₂Me | OCH₂CH₂ | NMePh | |
| 2.201 | Cl | SO₂Et | OCH₂CH₂ | NMePh | |
| 2.202 | Cl | Cl | OCH₂CH₂ | 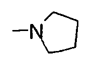 | |
| 2.203 | Br | Br | OCH₂CH₂ | 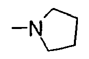 | |
| 2.204 | Cl | Br | OCH₂CH₂ | 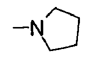 | |
| 2.205 | Me | Br | OCH₂CH₂ | 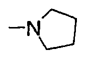 | |
| 2.206 | Cl | SO₂Me | OCH₂CH₂ | 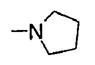 | |
| 2.207 | Me | SO₂Me | OCH₂CH₂ | 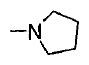 | |
| 2.208 | Cl | SO₂Et | OCH₂CH₂ | 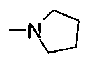 | |
| 2.209 | Cl | Cl | OCH₂CH=CH | NMe₂ | |
| 2.210 | Cl | SO₂Me | OCH₂CH=CH | NMe₂ | |
| 2.211 | Me | Cl | OCH₂CH=CH | NMe₂ | |
| 2.212 | Me | Br | OCH₂CH=CH | NMe₂ | |
| 2.213 | Me | SO₂Me | OCH₂CH=CH | NMe₂ | |
| 2.214 | Cl | Cl | OCH₂CH=CH | NEt₂ | |
| 2.215 | Cl | SO₂Me | OCH₂CH=CH | NEt₂ | |
| 2.216 | Me | Cl | OCH₂CH=CH | NEt₂ | |
| 2.217 | Me | Br | OCH₂CH=CH | NEt₂ | |
| 2.218 | Me | SO₂Me | OCH₂CH=CH | NEt₂ | |
| 2.219 | Cl | Cl | OCH₂CH=CH | Nh(c-Pr) | |
| 2.220 | Cl | SO₂Me | OCH₂CH=CH | Nh(c-Pr) | |
| 2.221 | Me | Cl | OCH₂CH=CH | Nh(c-Pr) | |
| 2.222 | Me | Br | OCH₂CH=CH | Nh(c-Pr) | |
| 2.223 | Me | SO₂M | OCH₂CH=CH | Nh(c-Pr) | |

**Tabelle 3: Erfindungsgemäße Verbindungen der Formel (I), worin die Substituenten und Symbole folgende Bedeutungen haben:**

| | | | | | |
|---|---|---|---|---|---|
| R^{1c} = H | | | R⁴ = Me | | R⁵ = Me |
| R⁶ = H | | | Y = O | | |
| 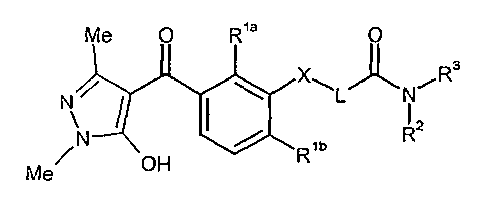 | | | | | |

| **Nr.** | **R^{1a}** | **R^{1b}** | **X-L** | **NR²R³** | **Physikal. Daten** |
|---|---|---|---|---|---|
| 3.1 | Cl | Cl | OCH₂ | NH₂ | |
| 3.2 | Br | Br | OCH₂ | NH₂ | |
| 3.3 | Me | Br | OCH₂ | NH₂ | |
| 3.4 | Cl | SO₂Me | OCH₂ | NH₂ | |
| 3.5 | Cl | SO₂Et | OCH₂ | NH₂ | |
| 3.6 | Me | SO₂Me | OCH₂ | NH₂ | |
| 3.6a | Me | Cl | OCH₂ | NHMe | |
| 3.6b | Me | Br | OCH₂ | NHMe | R^{f} (EE): 0,28 |
| 3.7 | Cl | Cl | OCH₂ | NHEt | |
| 3.8 | Cl | SO₂Me | OCH₂ | NHEt | |
| 3.8a | Me | Cl | OCH₂ | NHEt | |
| 3.8b | Me | Br | OCH₂ | NHEt | R^{f} (EE): 0,28 |
| 3.9 | Me | SO₂Me | OCH₂ | NHEt | |
| 3.9a | Me | Cl | OCH₂ | NH(Allyl) | |
| 3.9b | Me | Br | OCH₂ | NH(Allyl) | |
| 3.10 | Br | Br | OCH₂ | NH(i-Pr) | |
| 3.11 | Me | Br | OCH₂ | NH(i-Pr) | |
| 3.12 | Me | NO₂ | OCH₂ | NH(i-Pr) | |
| 3.13 | Cl | SO₂Et | OCH₂ | NH(i-Pr) | |
| 3.14 | Cl | Cl | OCH₂ | NH(c-Pr) | |
| 3.95 | Cl | Br | OCH₂ | NH(c-Pr) | |
| 3.15a | Me | Cl | OCH₂ | NH(c-Pr) | |
| 3.16 | Me | Br | OCH₂ | NH(c-Pr) | |
| 3.17 | Me | NO₂ | OCH₂ | NH(c-Pr) | |
| 3.18 | Cl | SO₂Me | OCH₂ | NH(c-Pr) | |
| 3.19 | Cl | Cl | OCH₂ | NMe₂ | |
| 3.20 | Br | Br | OCH₂ | NMe₂ | |
| 3.20a | Me | Cl | OCH₂ | NMe₂ | R^{f} (EE): 0,02 |
| 3.21 | Cl | Br | OCH₂ | NMe₂ | |
| 3.22 | Me | Br | OCH₂ | NMe₂ | |
| 3.23 | Cl | SO₂Me | OCH₂ | NMe₂ | |
| 3.24 | Me | SO₂Me | OCH₂ | NMe₂ | |
| 3.25 | Cl | SO₂Et | OCH₂ | NMe₂ | |
| 3.26 | Cl | Cl | OCH₂ | NEt₂ | |
| 3.27 | Br | Br | OCH₂ | NEt₂ | |
| 3.27a | Me | Cl | OCH₂ | NEt₂ | R^{f} (EE): 0,03 |
| 3.28 | Cl | Br | OCH₂ | NEt₂ | |
| 3.29 | Me | Br | OCH₂ | NEt₂ | |
| 3.30 | Cl | SO₂Me | OCH₂ | NEt₂ | |
| 3.31 | Me | SO₂Me | OCH₂ | NEt₂ | |
| 3.32 | Cl | SO₂Et | OCH₂ | NEt₂ | |
| 3.33 | Cl | Cl | OCH₂ | N(n-Pr)₂ | |
| 3.34 | Br | Br | OCH₂ | N(n-Pr)₂ | |
| 3.35 | Cl | Br | OCH₂ | N(n-Pr)₂ | |
| 3.36 | Me | Br | OCH₂ | N(n-Pr)₂ | |
| 3.37 | Cl | SO₂Me | OCH₂ | N(n-Pr)₂ | R^{f} (EE): 0,02 |
| 3.38 | Me | SO₂Me | OCH₂ | N(n-Pr)₂ | |
| 3.39 | Cl | SO₂Et | OCH₂ | N(n-Pr)₂ | |
| 3.40 | Cl | Cl | OCH₂ | N(i-Pr)₂ | |
| 3.41 | Me | Br | OCH₂ | N(i-Pr)₂ | |
| 3.42 | Me | NO₂ | OCH₂ | N(i-Pr)₂ | |
| 3.43 | NO₂ | Cl | OCH₂ | N(i-Pr)₂ | |
| 3.44 | NO₂ | Br | OCH₂ | N(i-Pr)₂ | |
| 3.45 | Me | SO₂Me | OCH₂ | N(i-Pr)₂ | |
| 3.46 | Cl | SO₂E | OCH₂ | N(i-Pr)₂ | |
| 3.47 | Cl | Cl | OCH₂ | NMePh | |
| 3.48 | Me | Br | OCH₂ | NMePh | |
| 3.49 | Me | NO₂ | OCH₂ | NMePh | |
| 3.50 | NO₂ | Cl | OCH₂ | NMePh | |
| 3.51 | NO₂ | Br | OCH₂ | NMePh | |
| 3.52 | Me | SO₂Me | OCH₂ | NMePh | |
| 3.53 | Cl | SO₂Et | OCH₂ | NMePh | |
| 3.54 | Cl | Cl | OCH₂ | 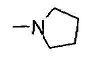 | |
| 3.55 | Br | Br | OCH₂ | 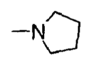 | R^{f} (EE): 0,01 |
| 3.56 | Cl | Br | OCH₂ | 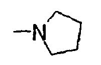 | |
| 3.57 | Me | Br | OCH₂ | 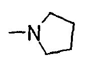 | |
| 3.58 | Cl | SO₂Me | OCH₂ | 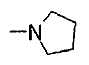 | |
| 3.59 | Me | SO₂Me | OCH₂ | 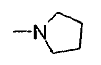 | |
| 3.60 | Cl | SO₂E | OCH₂ | 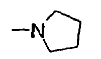 | |
| 3.61 | Cl | Cl | OCH₂ | 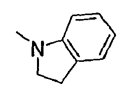 | |
| 3.62 | Br | Br | OCH₂ | 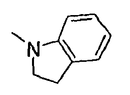 | |
| 3.63 | Cl | Br | OCH₂ | 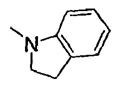 | |
| 3.64 | Me | Br | OCH₂ | 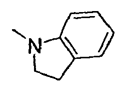 | |
| 3.65 | Cl | SO₂Me | OCH₂ | 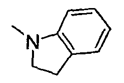 | |
| 3.66 | Me | SO₂Me | OCH₂ | 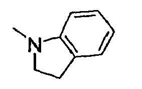 | |
| 3.67 | Cl | SO₂Et | OCH₂ | 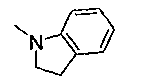 | |
| 3.68 | Cl | Cl | OCH₂ | 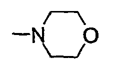 | |
| 3.69 | Me | Br | OCH₂ | 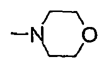 | |
| 3.70 | Me | NO₂ | OCH₂ | 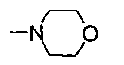 | |
| 3.71 | NO₂ | Cl | OCH₂ | 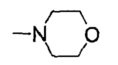 | |
| 3.72 | NO₂ | Br | OCH₂ | 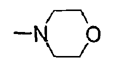 | |
| 3.73 | Me | SO₂Me | OCH₂ | 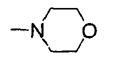 | |
| 3.74 | Cl | SO₂Et | OCH₂ | 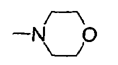 | |
| 3.75 | Cl | Cl | OCH(Me) | NH₂ | |
| 3.76 | Br | Br | OCH(Me) | NH₂ | |
| 3.77 | Me | Br | OCH(Me) | NH₂ | |
| 3.78 | Cl | SO₂Me | OCH(Me) | NH₂ | |
| 3.79 | Cl | SO₂Et | OCH(Me) | NH₂ | |
| 3.80 | Me | SO₂Me | OCH(Me) | NH₂ | |
| 3.80a | Cl | Cl | OCH(Me) | NHMe | |
| 3.80b | Cl | SO₂Me | OCH(Me) | NHMe | |
| 3.80c | Me | Cl | OCH(Me) | NHMe | |
| 3.80d | Me | Br | OCH(Me) | NHMe | |
| 3.80e | Me | SO₂Me | OCH(Me) | NHMe | |
| 3.81 | Cl | Cl | OCH(Me) | NHEt | R^{f} (EE): 0,35 |
| 3.82 | Cl | SO₂Me | OCH(Me) | NHEt | |
| 3.82a | Me | Cl | OCH(Me) | NHEt | |
| 3.82b | Me | Br | OCH(Me) | NHEt | |
| 3.82c | Cl | Cl | OCH(Me) | NH(Allyl) | R^{f} (EE): 0,05 |
| 3.82d | Cl | SO₂Me | OCH(Me) | NH(Allyl) | |
| 3.82e | Me | Cl | OCH(Me) | NH(Allyl) | |
| 3.82f | Me | Br | OCH(Me) | NH(Allyl) | |
| 3.82g | Me | SO₂Me | OCH(Me) | NH(Allyl) | |
| 3.83 | Me | SO₂Me | OCH(Me) | NHEt | |
| 3.84 | Br | Br | OCH(Me) | NH(i-Pr) | |
| 3.85 | Me | Br | OCH(Me) | NH(i-Pr) | R^{f} (EE): 0,11 |
| 3.86 | Me | NO₂ | OCH(Me) | NH(i-Pr) | |
| 3.87 | Cl | SO₂Et | OCH(Me) | NH(i-Pr) | |
| 3.88 | Cl | Cl | OCH(Me) | NH(c-Pr) | R^{f} (EE): 0,10 |
| 3.89 | Cl | Br | OCH(Me) | NH(c-Pr) | |
| 3.90 | Me | Br | OCH(Me) | NH(c-Pr) | |
| 3.91 | Me | NO₂ | OCH(Me) | NH(c-Pr) | |
| 3.92 | Cl | SO₂Me | OCH(Me) | NH(c-Pr) | |
| 3.93 | Cl | Cl | OCH(Me) | NMe₂ | |
| 3.94 | Br | Br | OCH(Me) | NMe₂ | |
| 3.95 | Cl | Br | OCH(Me) | NMe₂ | |
| 3.96 | Me | Br | OCH(Me) | NMe₂ | R^{f} (EE): 018 |
| 3.97 | Cl | SO₂Me | OCH(Me) | NMe₂ | |
| 3.98 | Me | SO₂Me | OCH(Me) | NMe₂ | |
| 3.99 | Cl | SO₂Et | OCH(Me) | NMe₂ | |
| 3.100 | Cl | Cl | OCH(Me) | NEt₂ | |
| 3.101 | Br | Br | OCH(Me) | NEt₂ | |
| 3.102 | Cl | Br | OCH(Me) | NEt₂ | |
| 3.103 | Me | Br | OCH(Me) | NEt₂ | |
| 3.104 | Cl | SO₂Me | OCH(Me) | NEt₂ | |
| 3.105 | Me | SO₂Me | OCH(Me) | NEt₂ | |
| 3.106 | Cl | SO₂Et | OCH(Me) | NEt₂ | |
| 3.107 | Cl | Cl | OCH(Me) | N(n-Pr)₂ | |
| 3.108 | Br | Br | OCH(Me) | N(n-Pr)₂ | |
| 3.109 | Cl | Br | OCH(Me) | N(n-Pr)₂ | |
| 3.110 | Me | Br | OCH(Me) | N(n-Pr)₂ | |
| 3.111 | Cl | SO₂Me | OCH(Me) | N(n-Pr)₂ | |
| 3.112 | Me | SO₂Me | OCH(Me) | N(n-Pr)₂ | |
| 3.113 | Cl | SO₂Et | OCH(Me) | N(n-Pr)₂ | |
| 3.114 | Cl | Cl | OCH(Me) | N(i-Pr)₂ | |
| 3.115 | Me | Br | OCH(Me) | N(i-Pr)₂ | |
| 3.116 | Me | NO₂ | OCH(Me) | N(i-Pr)₂ | |
| 3.117 | NO₂ | Cl | OCH(Me) | N(i-Pr)₂ | |
| 3.118 | NO₂ | Br | OCH(Me) | N(i-Pr)₂ | |
| 3.119 | Me | SO₂Me | OCH(Me) | N(i-Pr)₂ | |
| 3.120 | Cl | SO₂Et | OCH(Me) | N(i-Pr)₂ | |
| 3.121 | Cl | Cl | OCH(Me) | NMePh | |
| 3.122 | Me | Br | OCH(Me) | NMePh | |
| 3.123 | Me | NO₂ | OCH(Me) | NMePh | |
| 3.124 | NO₂ | Cl | OCH(Me) | NMePh | |
| 3.125 | NO₂ | Br | OCH(Me) | NMePh | |
| 3.126 | Me | SO₂Me | OCH(Me) | NMePh | |
| 3.127 | Cl | SO₂Et | OCH(Me) | NMePh | |
| 3.128 | Cl | Cl | OCH(Me) | 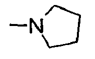 | |
| 3.129 | Br | Br | OCH(Me) | 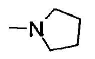 | |
| 3.130 | Cl | Br | OCH(Me) | 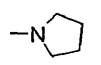 | |
| 3.131 | Me | Br | OCH(Me) | 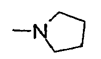 | |
| 3.132 | Cl | SO₂Me | OCH(Me) | 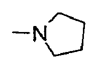 | |
| 3.133 | Me | SO₂Me | OCH(Me) | 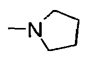 | |
| 3.134 | Cl | SO₂Et | OCH(Me) | 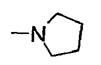 | |
| 3.135 | Cl | Cl | OCH(Me) | 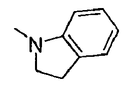 | |
| 3.136 | Br | Br | OCH(Me) | 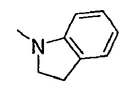 | |
| 3.137 | Cl | Br | OCH(Me) | 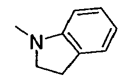 | |
| 3.138 | Me | Br | OCH(Me) | 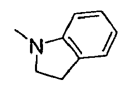 | |
| 3.139 | Cl | SO₂Me | OCH(Me) | 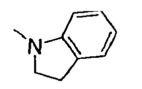 | |
| 3.140 | Me | SO₂Me | OCH(Me) | 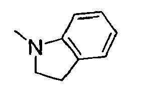 | |
| 3.141 | Cl | SO₂Et | OCH(Me) | 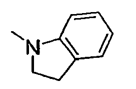 | |
| 3.142 | Cl | Cl | OCH(Me) | 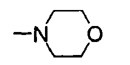 | |
| 3.143 | Me | Br | OCH(Me) | 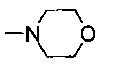 | |
| 3.144 | Me | NO₂ | OCH(Me) | 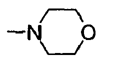 | |
| 3.145 | NO₂ | Cl | OCH(Me) | 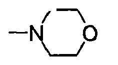 | |
| 3.146 | NO₂ | Br | OCH(Me) | 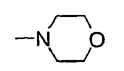 | |
| 3.147 | Me | SO₂M | OCH(Me) | 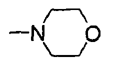 | |
| 3.148 | Cl | SO₂Et | OCH(Me) | 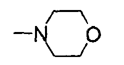 | |
| 3.149 | Cl | Cl | OCH₂CH₂ | NH₂ | |
| 3.150 | Br | Br | OCH₂CH₂ | NH₂ | |
| 3.151 | Me | Br | OCH₂CH₂ | NH₂ | |
| 3.152 | Cl | SO₂Me | OCH₂CH₂ | NH₂ | |
| 3.153 | Cl | SO₂Et | OCH₂CH₂ | NH₂ | |
| 3.154 | Me | SO₂Me | OCH₂CH₂ | NH₂ | |
| 3.155 | Cl | Cl | OCH₂CH₂ | NHEt | |
| 3.156 | Cl | SO₂Me | OCH₂CH₂ | NHEt | |
| 3.157 | Me | SO₂Me | OCH₂CH₂ | NHEt | |
| 3.158 | Br | Br | OCH₂CH₂ | NH(i-Pr) | |
| 3.159 | Me | Br | OCH₂CH₂ | NH(i-Pr) | |
| 3.160 | Me | NO₂ | OCH₂CH₂ | NH(i-Pr) | |
| 3.161 | Cl | SO₂Et | OCH₂CH₂ | NH(i-Pr) | |
| 3.162 | Cl | Cl | OCH₂CH₂ | NH(c-Pr) | |
| 3.163 | Cl | Br | OCH₂CH₂ | NH(c-Pr) | |
| 3.164 | Me | Br | OCH₂CH₂ | NH(c-Pr) | |
| 3.165 | Me | NO₂ | OCH₂CH₂ | NH(c-Pr) | |
| 3.166 | Cl | SO₂Me | OCH₂CH₂ | NH(c-Pr) | |
| 3.167 | Cl | Cl | OCH₂CH₂ | NMe₂ | |
| 3.168 | Br | Br | OCH₂CH₂ | NMe₂ | |
| 3.169 | Cl | Br | OCH₂CH₂ | NMe₂ | |
| 3.170 | Me | Br | OCH₂CH₂ | NMe₂ | |
| 3.171 | Cl | SO₂Me | OCH₂CH₂ | NMe₂ | |
| 3.172 | Me | SO₂Me | OCH₂CH₂ | NMe₂ | |
| 3.173 | Cl | SO₂Et | OCH₂CH₂ | NMe₂ | |
| 3.174 | Cl | Cl | OCH₂CH₂ | NEt₂ | |
| 3.175 | Br | Br | OCH₂CH₂ | NEt₂ | |
| 3.176 | Cl | Br | OCH₂CH₂ | NEt₂ | |
| 3.177 | Me | Br | OCH₂CH₂ | NEt₂ | |
| 3.178 | Cl | SO₂Me | OCH₂CH₂ | NEt₂ | |
| 3.179 | Me | SO₂Me | OCH₂CH₂ | NEt₂ | |
| 3.180 | Cl | SO₂Et | OCH₂CH₂ | NEt₂ | |
| 3.181 | Cl | Cl | OCH₂CH₂ | N(n-Pr)₂ | |
| 3.182 | Br | Br | OCH₂CH₂ | N(n-Pr)₂ | |
| 3.183 | Cl | Br | OCH₂CH₂ | N(n-Pr)₂ | |
| 3.184 | Me | Br | OCH₂CH₂ | N(n-Pr)₂ | |
| 3.185 | Cl | SO₂Me | OCH₂CH₂ | N(n-Pr)₂ | |
| 3.186 | Me | SO₂Me | OCH₂CH₂ | N(n-Pr)₂ | |
| 3.187 | Cl | SO₂Et | OCH₂CH₂ | N(n-Pr)₂ | |
| 3.188 | Cl | Cl | OCH₂CH₂ | N(i-Pr)₂ | |
| 3.189 | Me | Br | OCH₂CH₂ | N(i-Pr)₂ | |
| 3.190 | Me | NO₂ | OCH₂CH₂ | N(i-Pr)₂ | |
| 3.191 | NO₂ | Cl | OCH₂CH₂ | N(i-Pr)₂ | |
| 3.192 | NO₂ | Br | OCH₂CH₂ | N(i-Pr)₂ | |
| 3.193 | Me | SO₂Me | OCH₂CH₂ | N(i-Pr)₂ | |
| 3.194 | Cl | SO₂Et | OCH₂CH₂ | N(i-Pr)₂ | |
| 3.195 | Cl | Cl | OCH₂CH₂ | NMePh | |
| 3.196 | Me | Br | OCH₂CH₂ | NMePh | |
| 3.197 | Me | NO₂ | OCH₂CH₂ | NMePh | |
| 3.198 | NO₂ | Cl | OCH₂CH₂ | NMePh | |
| 3.199 | NO₂ | Br | OCH₂CH₂ | NMePh | |
| 3.200 | Me | SO₂Me | OCH₂CH₂ | NMePh | |
| 3.201 | Cl | SO₂Et | OCH₂CH₂ | NMePh | |
| 3.202 | Cl | Cl | OCH₂CH₂ | 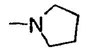 | |
| 3.203 | Br | Br | OCH₂CH₂ | 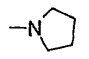 | |
| 3.204 | Cl | Br | OCH₂CH₂ | 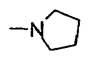 | |
| 3.205 | Me | Br | OCH₂CH₂ | 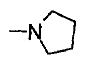 | |
| 3.206 | Cl | SO₂Me | OCH₂CH₂ | 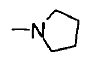 | |
| 3.207 | Me | SO₂Me | OCH₂CH₂ | 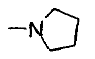 | |
| 3.208 | Cl | SO₂Et | OCH₂CH₂ | 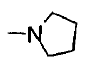 | |
| 3.209 | Cl | Cl | OCH₂CH=CH | NMe₂ | |
| 3.210 | Cl | SO₂M | OCH₂CH=CH | NMe₂ | |
| 3.211 | Me | Cl | OCH₂CH=CH | NMe₂ | |
| 3.212 | Me | Br | OCH₂CH=CH | NMe₂ | |
| 3.213 | Me | SO₂Me | OCH₂CH=CH | NMe₂ | |
| 3.214 | Cl | Cl | OCH₂CH=CH | NEt₂ | |
| 3.215 | Cl | SO₂Me | OCH₂CH=CH | NEt₂ | |
| 3.216 | Me | Cl | OCH₂CH=CH | NEt₂ | |
| 3.217 | Me | Br | OCH₂CH=CH | NEt₂ | |
| 3.218 | Me | SO₂Me | OCH₂CH=CH | NEt₂ | |
| 3.219 | Cl | Cl | OCH₂CH=CH | Nh(c-Pr) | |
| 3.220 | Cl | SO₂Me | OCH,-CH=CH | Nh(c-Pr) | |
| 3.221 | Me | Cl | OCH₂H=CH | Nh(c-Pr) | |
| 3.222 | Me | Br | OCH₂CH=CH | Nh(c-Pr) | |
| 3.223 | Me | SO₂Me | OCH₂CH=CH | Nh(c-Pr) | |

**Tabelle 4: Erfindungsgemäße Verbindungen der Formel (I), worin die Substituenten und Symbole folgende Bedeutungen haben:**

| | | | | | |
|---|---|---|---|---|---|
| R^{1c} = H | | | R⁴ = c-Pr | | R⁵ = Me |
| R⁶ = H | | | Y = O | | |
| 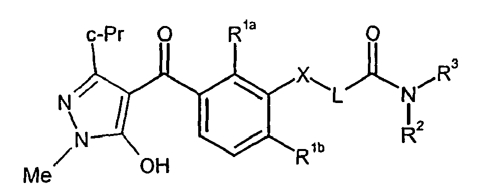 | | | | | |

| **Nr.** | **R^{1a}** | **R^{1b}** | **X-L** | **NR²R³** | **Physikal. Daten** |
|---|---|---|---|---|---|
| 4.1 | Cl | Cl | OCH₂ | NH₂ | |
| 4.2 | Br | Br | OCH₂ | NH₂ | |
| 4.3 | Me | Br | OCH₂ | NH₂ | |
| 4.4 | Cl | SO₂Me | OCH₂ | NH₂ | |
| 4.5 | Cl | SO₂Et | OCH₂ | NH₂ | |
| 4.6 | Me | SO₂Me | OCH₂ | NH₂ | |
| 4.6a | Me | Cl | OCH₂ | NHMe | |
| 4.6b | Me | Br | OCH₂ | NHMe | |
| 4.7 | Cl | Cl | OCH₂ | NHEt | |
| 4.8 | Cl | SO₂Me | OCH₂ | NHEt | |
| 4.8a | Me | Cl | OCH₂ | NHEt | |
| 4.8b | Me | Br | OCH₂ | NHEt | |
| 4.9 | Me | SO₂Me | OCH₂ | NHEt | |
| 4.10 | Br | Br | OCH₂ | NH(i-Pr) | |
| 4.11 | Me | Br | OCH₂ | NH(i-Pr) | |
| 4.12 | Me | NO₂ | OCH₂ | NH(i-Pr) | |
| 4.13 | Cl | SO₂Et | OCH₂ | NH(i-Pr) | |
| 4.14 | Cl | Cl | OCH₂ | NH(c-Pr) | |
| 4.15 | Cl | Br | OCH₂ | NH(c-Pr) | |
| 4.15a | Me | Cl | OCH₂ | NH(c-Pr) | |
| 4.16 | Me | Br | OCH₂ | NH(c-Pr) | |
| 4.17 | Me | NO₂ | OCH₂ | NH(c-Pr) | |
| 4.18 | Cl | SO₂Me | OCH₂ | NH(c-Pr) | |
| 4.19 | Cl | Cl | OCH₂ | NMe₂ | |
| 4.20 | Br | Br | OCH₂ | NMe₂ | |
| 4.20a | Me | Cl | OCH₂ | NMe₂ | R^{f} (EE): 0,04 |
| 4.21 | Cl | Br | OCH₂ | NMe₂ | |
| 4.22 | Me | Br | OCH₂ | NMe₂ | |
| 4.23 | Cl | SO₂Me | OCH₂ | NMe₂ | |
| 4.24 | Me | SO₂Me | OCH₂ | NMe₂ | |
| 4.25 | Cl | SO₂Et | OCH₂ | NMe₂ | |
| 4.26 | Cl | Cl | OCH₂ | NEt₂ | |
| 4.27 | Br | Br | OCH₂ | NEt₂ | |
| 4.27a | Me | Cl | OCH₂ | NEt₂ | R^{f} (EE): 0,06 |
| 4.28 | Cl | Br | OCH₂ | NEt₂ | |
| 4.29 | Me | Br | OCH₂ | NEt₂ | |
| 4.30 | Cl | SO₂Me | OCH₂ | NEt₂ | |
| 4.31 | Me | SO₂Me | OCH₂ | NEt₂ | |
| 4.32 | Cl | SO₂Et | OCH₂ | NEt₂ | |
| 4.33 | Cl | Cl | OCH₂ | N(n-Pr)₂ | |
| 4.34 | Br | Br | OCH₂ | N(n-Pr)₂ | |
| 4.35 | Cl | Br | OCH₂ | N(n-Pr)₂ | |
| 4.36 | Me | Br | OCH₂ | N(n-Pr)₂ | |
| 4.37 | Cl | SO₂Me | OCH₂ | N(n-Pr)₂ | |
| 4.38 | Me | SO₂Me | OCH₂ | N(n-Pr)₂ | |
| 4.39 | Cl | SO₂Et | OCH₂ | N(n-Pr)₂ | |
| 4.40 | Cl | Cl | OCH₂ | N(i-Pr)₂ | |
| 4.41 | Me | Br | OCH₂ | N(i-Pr)₂ | |
| 4.42 | Me | NO₂ | OCH₂ | N(i-Pr)₂ | |
| 4.43 | NO₂ | Cl | OCH₂ | N(i-Pr)₂ | |
| 4.44 | NO₂ | Br | OCH₂ | N(i-Pr)₂ | |
| 4.45 | Me | SO₂Me | OCH₂ | N(i-Pr)₂ | |
| 4.46 | Cl | SO₂Et | OCH₂ | N(i-Pr)₂ | |
| 4.47 | Cl | Cl | OCH₂ | NMePh | |
| 4.48 | Me | Br | OCH₂ | NMePh | |
| 4.49 | Me | NO₂ | OCH₂ | NMePh | |
| 4.50 | NO₂ | Cl | OCH₂ | NMePh | |
| 4.51 | NO₂ | Br | OCH₂ | NMePh | |
| 4.52 | Me | SO₂Me | OCH₂ | NMePh | |
| 4.53 | Cl | SO₂Et | OCH₂ | NMePh | |
| 4.54 | Cl | Cl | OCH₂ | 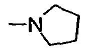 | |
| 4.55 | Br | Br | OCH₂ | 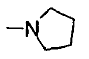 | |
| 4.56 | Cl | Br | OCH₂ | 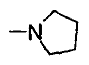 | |
| 4.57 | Me | Br | OCH₂ | 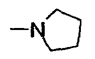 | |
| 4.58 | Cl | SO₂Me | OCH₂ | 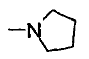 | |
| 4.59 | Me | SO₂Me | OCH₂ | 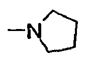 | |
| 4.60 | Cl | SO₂Et | OCH₂ | 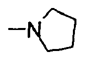 | |
| 4.61 | Cl | Cl | OCH₂ | 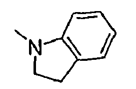 | |
| 4.62 | Br | Br | OCH₂ | 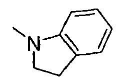 | |
| 4.63 | Cl | Br | OCH₂ | 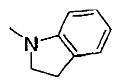 | |
| 4.64 | Me | Br | OCH₂ | 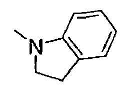 | |
| 4.65 | Cl | SO₂Me | OCH₂ | 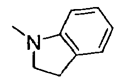 | |
| 4.66 | Me | SO₂Me | OCH₂ | 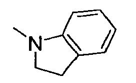 | |
| 4.67 | Cl | SO₂Et | OCH₂ | 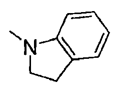 | |
| 4.68 | Cl | Cl | OCH₂ | 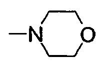 | |
| 4.69 | Me | Br | OCH₂ | 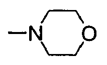 | |
| 4.70 | Me | NO₂ | OCH₂ | 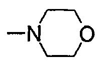 | |
| 4.71 | NO₂ | Cl | OCH₂ | 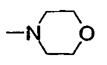 | |
| 4.72 | NO₂ | Br | OCH₂ | 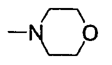 | |
| 4.73 | Me | SO₂Me | OCH₂ | 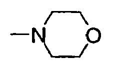 | |
| 4.74 | Cl | SO₂Et | OCH₂ | 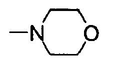 | |
| 4.75 | Cl | Cl | OCH(Me) | NH₂ | |
| 4.76 | Br | Br | OCH(Me) | NH₂ | |
| 4.77 | Me | Br | OCH(Me) | NH₂ | |
| 4.78 | Cl | SO₂Me | OCH(Me) | NH₂ | |
| 4.79 | Cl | SO₂Et | OCH(Me) | NH₂ | |
| 4.80 | Me | SO₂Me | OCH(Me) | NH₂ | |
| 4.80a | Cl | Cl | OCH(Me) | NHMe | |
| 4.80b | Cl | SO₂Me | OCH(Me) | NHMe | |
| 4.80c | Me | Cl | OCH(Me) | NHMe | |
| 4.80d | Me | Br | OCH(Me) | NHMe | |
| 4.80e | Me | SO₂Me | OCH(Me) | NHMe | |
| 4.81 | Cl | Cl | OCH(Me) | NHEt | |
| 4.82 | Cl | SO₂Me | OCH(Me) | NHEt | |
| 4.82a | Me | Cl | OCH(Me) | NHEt | |
| 4.82b | Me | Br | OCH(Me) | NHEt | |
| 4.82c | Cl | Cl | OCH(Me) | NH(Allyl) | |
| 4.82d | Cl | SO₂Me | OCH(Me) | NH(Allyl) | |
| 4.82e | Me | Cl | OCH(Me) | NH(Allyl) | |
| 4.82f | Me | Br | OCH(Me) | NH(Allyl) | |
| 4.82g | Me | SO₂Me | OCH(Me) | NH(Allyl) | |
| 4.83 | Me | SO₂Me | OCH(Me) | NHEt | |
| 4.84 | Br | Br | OCH(Me) | NH(i-Pr) | |
| 4.85 | Me | Br | OCH(Me) | NH(i-Pr) | |
| 4.86 | Me | NO₂ | OCH(Me) | NH(i-Pr) | |
| 4.87 | Cl | SO₂Et | OCH(Me) | NH(i-Pr) | |
| 4.88 | Cl | Cl | OCH(Me) | NH(c-Pr) | |
| 4.89 | Cl | Br | OCH(Me) | NH(c-Pr) | |
| 4.90 | Me | Br | OCH(Me) | NH(c-Pr) | |
| 4.91 | Me | NO₂ | OCH(Me) | NH(c-Pr) | |
| 4.92 | Cl | SO₂Me | OCH(Me) | NH(c-Pr) | |
| 4.93 | Cl | Cl | OCH(Me) | NMe₂ | |
| 4.94 | Br | Br | OCH(Me) | NMe₂ | |
| 4.95 | Cl | Br | OCH(Me) | NMe₂ | |
| 4.96 | Me | Br | OCH(Me) | NMe₂ | R^{f} (EE): 0,01 |
| 4.97 | Cl | SO₂Me | OCH(Me) | NMe₂ | |
| 4.98 | Me | SO₂Me | OCH(Me) | NMe₂ | |
| 4.99 | Cl | SO₂Et | OCH(Me) | NMe₂ | |
| 4.100 | Cl | Cl | OCH(Me) | NEt₂ | |
| 4.101 | Br | Br | OCH(Me) | NEt₂ | |
| 4.102 | Cl | Br | OCH(Me) | NEt₂ | |
| 4.103 | Me | Br | OCH(Me) | NEt₂ | |
| 4.104 | Cl | SO₂Me | OCH(Me) | NEt₂ | |
| 4.105 | Me | SO₂Me | OCH(Me) | NEt₂ | |
| 4.106 | Cl | SO₂Et | OCH(Me) | NEt₂ | |
| 4.107 | Cl | Cl | OCH(Me) | N(n-Pr)₂ | |
| 4.108 | Br | Br | OCH(Me) | N(n-Pr)₂ | |
| 4.109 | Cl | Br | OCH(Me) | N(n-Pr)₂ | |
| 4.110 | Me | Br | OCH(Me) | N(n-Pr)₂ | |
| 4.111 1 | Cl | SO₂Me | OCH(Me) | N(n-Pr)₂ | |
| 4.112 | Me | SO₂Me | OCH(Me) | N(n-Pr)₂ | |
| 4.113 | Cl | SO₂Et | OCH(Me) | N(n-Pr)₂ | |
| 4.114 | Cl | Cl | OCH(Me) | N(i-Pr)₂ | |
| 4.115 | Me | Br | OCH(Me) | N(i-Pr)₂ | |
| 4.116 | Me | NO₂ | OCH(Me) | N(i-Pr)₂ | |
| 4.117 | NO₂ | Cl | OCH(Me) | N(i-Pr)₂ | |
| 4.118 | NO₂ | Br | OCH(Me) | N(i-Pr)₂ | |
| 4.119 | Me | SO₂Me | OCH(Me) | N(i-Pr)₂ | |
| 4.120 | Cl | SO₂E | OCH(Me) | N(i-Pr)₂ | |
| 4.121 | Cl | Cl | OCH(Me) | NMePh | |
| 4.122 | Me | Br | OCH(Me) | NMePh | |
| 4.123 | Me | NO₂ | OCH(Me) | NMePh | |
| 4.124 | NO₂ | Cl | OCH(Me) | NMePh | |
| 4.125 | NO₂ | Br | OCH(Me) | NMePh | |
| 4.126 | Me | SO₂M | OCH(Me) | NMePh | |
| 4.127 | Cl | SO₂E | OCH(Me) | NMePh | |
| 4.128 | Cl | Cl | OCH(Me) | 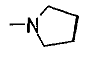 | |
| 4.129 | Br | Br | OCH(Me) | 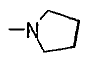 | |
| 4.130 | Cl | Br | OCH(Me) | 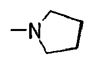 | |
| 4.131 | Me | Br | OCH(Me) | 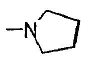 | |
| 4.132 | Cl | SO₂Me | OCH(Me) | 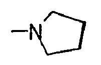 | |
| 4.133 | Me | SO₂Me | OCH(Me) | 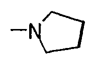 | |
| 4.134 | Cl | SO₂Et | OCH(Me) | 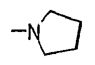 | |
| 4.135 | Cl | Cl | OCH(Me) | 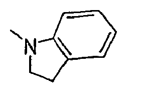 | |
| 4.136 | Br | Br | OCH(Me) | 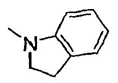 | |
| 4.137 | Cl | Br | OCH(Me) | 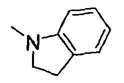 | |
| 4.138 | Me | Br | OCH(Me) | 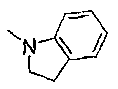 | |
| 4.139 | Cl | SO₂Me | OCH(Me) | 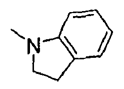 | |
| 4.140 | Me | SO₂Me | OCH(Me) | 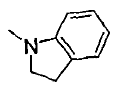 | |
| 4.141 | Cl | SO₂Et | OCH(Me) | 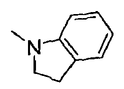 | |
| 4.142 | Cl | Cl | OCH(Me) | 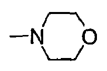 | |
| 4.143 | Me | Br | OCH(Me) | 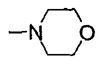 | |
| 4.144 | Me | NO₂ | OCH(Me) | 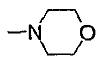 | |
| 4.145 | NO₂ | Cl | OCH(Me) | 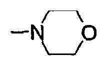 | |
| 4.146 | NO₂ | Br | OCH(Me) | 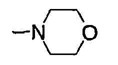 | |
| 4.147 | Me | SO₂Me | OCH(Me) | 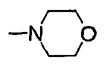 | |
| 4.148 | Cl | SO₂Et | OCH(Me) | 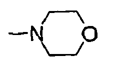 | |
| 4.149 | Cl | Cl | OCH₂CH₂ | NH₂ | |
| 4.150 | Br | Br | OCH₂CH₂ | NH₂ | |
| 4.151 | Me | Br | OCH₂CH₂ | NH₂ | |
| 4.152 | Cl | SO₂Me | OCH₂CH₂ | NH₂ | |
| 4.153 | Cl | SO₂Et | OCH₂CH₂ | NH₂ | |
| 4.154 | Me | SO₂Me | OCH₂CH₂ | NH₂ | |
| 4.155 | Cl | Cl | OCH₂CH₂ | NHEt | |
| 4.156 | Cl | SO₂Me | OCH₂CH₂ | NHEt | |
| 4.157 | Me | SO₂Mee | OCH₂CH₂ | NHEt | |
| 4.158 | Br | Br | OCH₂CH₂ | NH(i-Pr) | |
| 4.159 | Me | Br | OCH₂CH₂ | NH(i-Pr) | |
| 4.160 | Me | NO₂ | OCH₂CH₂ | NH(i-Pr) | |
| 4.161 | Cl | SO₂ Et | OCH₂CH₂ | NH(i-Pr) | |
| 4.162 | Cl | Cl | OCH₂CH₂ | NH(c-Pr) | |
| 4.163 | Cl | Br | OCH₂CH₂ | NH(c-Pr) | |
| 4.164 | Me | Br | OCH₂CH₂ | NH(c-Pr) | |
| 4.165 | Me | NO₂ | OCH₂CH₂ | NH(c-Pr) | |
| 4.166 | Cl | SO₂Me | OCH₂CH₂ | NH(c-Pr) | |
| 4.167 | Cl | Cl | OCH₂CH₂ | NMe₂ | |
| 4.168 | Br | Br | OCH₂CH₂ | NMe₂ | |
| 4.169 | Cl | Br | OCH₂CH₂ | NMe₂ | |
| 4.170 | Me | Br | OCH₂CH₂ | NMe₂ | |
| 4.171 | Cl | SO₂Me | OCH₂CH₂ | NMe₂ | |
| 4.172 | Me | SO₂Me | OCH₂CH₂ | NMe₂ | |
| 4.173 | Cl | SO₂Et | OCH₂CH₂ | NMe₂ | |
| 4.174 | Cl | Cl | OCH₂CH₂ | NEt₂ | |
| 4.175 | Br | Br | OCH₂CH₂ | NEt₂ | |
| 4.176 | Cl | Br | OCH₂CH₂ | NEt₂ | |
| 4.177 | Me | Br | OCH₂CH₂ | NEt₂ | |
| 4.178 | Cl | SO₂Me | OCH₂CH₂ | NEt₂ | |
| 4.179 | Me | SO₂Me | OCH₂CH₂ | NEt₂ | |
| 4.180 | Cl | SO₂Et | OCH₂CH₂ | NEt₂ | |
| 4.181 | Cl | Cl | OCH₂CH₂ | N(n-Pr)₂ | |
| 4.182 | Br | Br | OCH₂CH₂ | N(n-Pr)₂ | |
| 4.183 | Cl | Br | OCH₂CH₂ | N(n-Pr)₂ | |
| 4.184 | Me | Br | OCH₂CH₂ | N(n-Pr)₂ | |
| 4.185 | Cl | SO₂Me | OCH₂CH₂ | N(n-Pr)₂ | |
| 4.186 | Me | SO₂Me | OCH₂CH₂ | N(n-Pr)₂ | |
| 4.187 | Cl | SO₂Et | OCH₂CH₂ | N(n-Pr)₂ | |
| 4.188 | Cl | Cl | OCH₂CH₂ | N(i-Pr)₂ | |
| 4.189 | Me | Br | OCH₂CH₂ | N(i-Pr)₂ | |
| 4.190 | Me | NO₂ | OCH₂CH₂ | N(i-Pr)₂ | |
| 4.191 | NO₂ | Cl | OCH₂CH₂ | N(i-Pr)₂ | |
| 4.192 | NO₂ | Br | OCH₂CH₂ | N(i-Pr)₂ | |
| 4.193 | Me | SO₂Me | OCH₂CH₂ | N(i-Pr)₂ | |
| 4.194 | Cl | SO₂Et | OCH₂CH₂ | N(i-Pr)₂ | |
| 4.195 | Cl | Cl | OCH₂CH₂ | NMePh | |
| 4.196 | Me | Br | OCH₂CH₂ | NMePh | |
| 4.197 | Me | NO₂ | OCH₂CH₂ | NMePh | |
| 4.198 | NO₂ | Cl | OCH₂CH₂ | NMePh | |
| 4.199 | NO₂ | Br | OCH₂CH₂ | NMePh | |
| 4.200 | Me | SO₂Me | OCH₂CH₂ | NMePh | |
| 4.201 | Cl | SO₂Et | OCH₂CH₂ | NMePh | |
| 4.202 | Cl | Cl | OCH₂CH₂ | 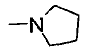 | |
| 4.203 | Br | Br | OCH₂CH₂ | 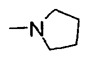 | |
| 4.204 | Cl | Br | OCH₂CH₂ | 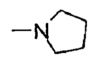 | |
| 4.205 | Me | Br | OCH₂CH₂ | 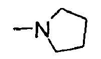 | |
| 4.206 | Cl | SO₂Me | OCH₂CH₂ | 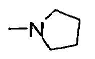 | |
| 4.207 | Me | SO₂Me | OCH₂CH₂ | 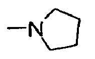 | |
| 4.208 | Cl | SO₂Et | OCH₂CH₂ | 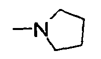 | |
| 4.209 | Cl | Cl | OCH₂CH=CH | NMe₂ | |
| 4.210 | Cl | SO₂Me | OCH₂CH=CH | NMe₂ | |
| 4.211 | Me | Cl | OCH₂CH=CH | NMe₂ | |
| 4.212 | Me | Br | OCH₂CH=CH | NMe₂ | |
| 4.213 | Me | SO₂Me | OCH₂CH=CH | NMe₂ | |
| 4.214 | Cl | Cl | OCH₂CH=CH | NEt₂ | |
| 4.215 | Cl | SO₂M | OCH₂CH=CH | NEt₂ | |
| 4.216 | Me | Cl | OCH₂CH=CH | NEt₂ | |
| 4.217 | Me | Br | OCH₂CH=CH | NEt₂ | |
| 4.218 | Me | SO₂Me | OCH₂CH=CH | NEt₂ | |
| 4.219 | Cl | Cl | OCH₂CH=CH | Nh(c-Pr) | |
| 4.220 | Cl | SO₂Mee | OCH₂CH=CH | Nh(c-Pr) | |
| 4.221 | Me | Cl | OCH₂CH=CH | Nh(c-Pr) | |
| 4.222 | Me | Br | OCH₂CH=CH | Nh(c-Pr) | |
| 4.223 | Me | SO₂Me | OCH₂CH=CH | Nh(c-Pr) | |

**Tabelle 5: Erfindungsgemäße Verbindungen der Formel (I), worin die Substituenten und Symbole folgende Bedeutungen haben:**

| | | | | | | | | |
|---|---|---|---|---|---|---|---|---|
| R^{1c} = H | | | | | R⁴ = Me | | R⁶ = c-Pr | |
| Y = O | | | | | | | | |
| 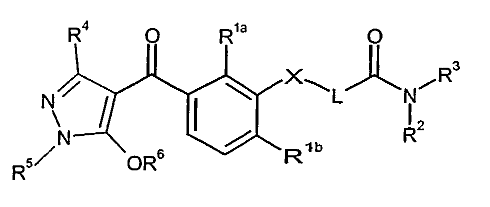 | | | | | | | | |

| **Nr.** | **R^{1a}** | **R^{1b}** | **R⁴** | **R⁵** | **R⁵** | **X-L** | **NR²R³** | **Physikal. Daten** |
|---|---|---|---|---|---|---|---|---|
| 5.1 | Cl | Cl | H | Me | Bz | OCH₂ | NEt₂ | |
| 5.2 | Cl | SO₂Et | H | Me | SO₂-(n-Pr) | OCH₂ | 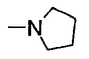 | |
| 5.3 | Me | Br | H | Me | CH₂-(2,6-F₂-Ph) | OCH₂ | NMe₂ | |
| 5.4 | Cl | Cl | H | Me | CH₂-Bz | OCH₂ | NH(c-Pr) | |
| 5.5 | Cl | Cl | H | Et | Bz | OCH₂ | NEt₂ | |
| 5.6 | Cl | SO₂Et | H | Et | SO₂-(n-Pr) | OCH₂ | 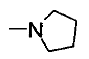 | |
| 5.7 | Me | Br | H | Et | CH₂-(2,6-F₂-Ph) | OCH₂ | NMe₂ | |
| 5.8 | Cl | Cl | H | Et | CH₂-Bz | OCH₂ | NH(c-Pr) | |
| 5.9 | Cl | Cl | Me | Me | Bz | OCH₂ | NEt₂ | |
| 5.10 | Cl | SO₂Et | Me | Me | SO₂-(n-Pr) | OCH₂ | 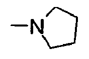 | |
| 5.11 | Me | Br | Me | Me | CH₂-(2,6-F₂-Ph) | OCH₂ | NMe₂ | |
| 5.12 | Cl | Cl | Me | Me | CH₂-Bz | OCH₂ | NH(c-Pr) | |
| 5.13 | Cl | Cl | H | Me | Bz | OC(Me)H | NEt₂ | |
| 5.14 | Cl | SO₂Et | H | Me | SO₂-(n-Pr) | OC(Me)H | 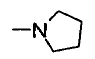 | |
| 5.15 | Me | Br | H | Me | CH₂-(2,6-F₂-Ph) | OC(Me)H | NMe₂ | |
| 5.16 | Cl | Cl | H | Me | CH₂-Bz | OC(Me)H | NH(c-Pr) | |
| 5.17 | Cl | Cl | H | Et | Bz | OC(Me)H | NEt₂ | |
| 5.18 | Cl | SO₂Et | H | Et | SO₂-(n-Pr) | OC(Me)H | 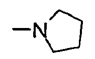 | |
| 5.19 | Me | Br | H | Et | CH₂-(2,6-F₂-Ph) | OC(Me)H | NMe₂ | |
| 5.20 | Cl | Cl | H | Et | CH₂-Bz | OC(Me)H | NH(c-Pr) | |
| 5.21 | Cl | Cl | Me | Me | Bz | OC(Me)H | NEt₂ | |
| 5.22 | Cl | SO₂Et | Me | Me | SO₂-(n-Pr) | OC(Me)H | 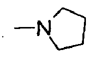 | |
| 5.23 | Me | Br | Me | Me | CH₂-(2,6-F₂-Ph) | OC(Me)H | NMe₂ | |
| 5.24 | Cl | Cl | Me | Me | CH₂-Bz | OC(Me)H | NH(c-Pr) | |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als, Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | |
|---|---|
| 75 | Gew.-Teile einer Verbindung der allgemeinen Formel(I), |
| 10 | " ligninsulfonsaures Calcium, |
| 5 | " Natriumlaurylsulfat, |
| 3 | " Polyvinylalkohol und |
| 7 | " Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | |
|---|---|
| 25 | Gew.-Teile einer Verbindung der allgemeinen Formel (I), |
| 5 | " 2,2'-dinaphthylmethan-6,6'-disulfonsaures Natrium, |
| 2 | " oleoylmethyltaurinsaures Natrium, |
| 1 | " Polyvinylalkohol, |
| 17 | " Calciumcarbonat und |
| 50 | " Wasser |

auf einer Kolloid mühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 I/ha in einer in den Tabellen 1 bis 5 angegebenen Dosierung auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen im Vergleich zu Verbindungen, die im Stand der Technik offenbart sind, bonitiert. Wie die Ergebnisse dieser Vergleichstabellen zeigen, weisen die ausgewählten erfindungsgemäßen Verbindungen dabei eine hervorragende Wirksamkeit gegen ein breites Spektrum wirtschaftlich wichtiger mono- und dikotyler Schadpflanzen auf.

### 2. Kulturpflanzenverträglichkeit

In weiteren Versuchen im Gewächshaus werden Samen von Gerste und mono- und dikotyler Schadpflanzen in sandigem Lehmboden ausgelegt, mit Erde abgedeckt und im Gewächshaus aufgestellt, bis die Pflanzen zwei bis drei echte Blätter entwickelt haben. Die Behandlung mit den erfindungsgemäßen Verbindungen der Formel (I) und im Vergleich dazu mit den im Stand der Technik offenbarten erfolgt dann wie oben unter Punkt 1 beschrieben. Vier bis fünf Wochen nach der Applikation und Standzeit im Gewächshaus wird mittels optischer Bonitur festgestellt, daß die erfindungsgemäßen Verbindungen eine hervorragende Verträglichkeit gegenüber wichtigen Kulturpflanzen, insbesondere Weizen, Mais und Reis, aufweisen.

## Patentansprüche

1. Verbindungen der Formel (I) oder deren Salze 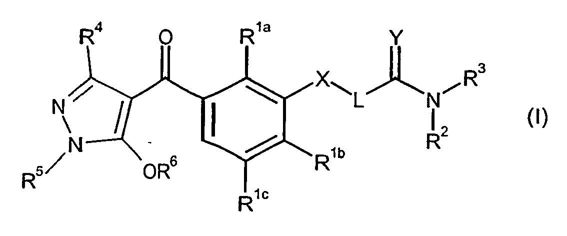 worin die Reste und Indizes folgende Bedeutungen haben:
X bedeutet O, S(O)ₙ, N-H oder N-R²;
L bedeutet eine geradkettige oder verzweigte durch w Reste aus der Gruppe Halogen, Cyano und Nitro und durch v Reste R² substituierte (C₁-C₆)-Alkylen-, (C₂-C₆)-Alkenylen- oder (C₂-C₆)-Alkinylenkette;
Y bedeutet Sauerstoff oder Schwefel;
R^{1a}, R^{1b}, R^{1c} bedeuten unabhängig voneinander Wasserstoff, Mercapto, Nitro, Halogen, Cyano, Thiocyanato, (C₁-C₆)-Alkyl-CO-O, (C₁-C₆)-Alkyl-S(O)n-O, (C₁-C₆)-Alkyl-S(O)ₘ, (C₁-C₆)-Haloalkyl-S(O)ₘ, (C₃-C₇)-Cycloalkyl-S(O)ₘ, Di-(C₁-C₆)-Alkyl-N-SO₂, (C₁-C₆)-Alkyl-SO₂-NH, (C₁-C₆)-Alkyl-NH-CO, Di-(C₁-C₆)-Alkyl-N-CO, (C₁-C₆)-Alkyl-SO₂-[(C₁-C₆)-alkyl]amino, (C₁-C₆)-Alkyl-CO-[(C₁-C₆)-alkyl]amino, (C₁-C₆)-Alkyl-O-CH₂, (C₁-C₆)-Alkyl-S(O)ₙ-CH₂, (C₁-C₆)-Alkyl-NH-CH₂, 1,2,4-Triazol-1-yl, 1,2,4-Triazol-1-yl-CH₂, jeweils durch v Reste aus der Gruppe Cyano, Nitro und Halogen substituiertes (C₁-C₆)-Alkyl-(Y)ₚ, (C₂-C₆)-Alkenyl-(Y)ₚ, (C₂-C₆)-Alkinyl-(Y)ₚ, (C₃-C₉)-Cycloalkyl-(Y)ₚ, (C₃-C₉)-Cyloalkenyl-(Y)ₚ, (C₁-C₆)-Alkyl-(C₃-C₉)-cycloalkyl-(Y)ₚ oder (C₁-C₆)-Alkyl-(C₃-C₉)-cycloalkenyl-(Y)ₚ;
R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉)-cycloalkyl, (C₁-C₆)-Alkyl-(C₃-C₉)-cycloalkenyl, (C₂-C₆)-Alkenyl-(C₃-C₉)-cycloalkyl, (C₂-C₆)-Alkenyl-(C₃-C₉)-cycloalkenyl, (C₂-C₆)-Alkinyl-(C₃-C₉)-cycloalkyl, (C₂-C₆)-Alkinyl-(C₃-C₉)-cycloalkenyl, geradkettiges oder verzweigtes [O-C(R⁶)₂]_{w}-[O-C(R⁶)-₂]ₓ-R⁶, (C₁-C₆)-Alkyl-aryl, (C₂-C₆)-Alkenyl-aryl, (C₂-C₆)-Alkinyl-aryl, geradkettiges oder verzweigtes [O-C(R⁶)₂]_{w}-[O-C(R⁶)₂]ₓ-Aryl, wobei die letzten 16 der vorstehend genannten Reste durch v Reste aus der Gruppe Cyano, Nitro und Halogen substituiert sind,
jeweils durch v Reste aus der Gruppe Cyano, Nitro, Halogen, (C₁-C₆)-Alkyl-(Y)ₚ und Halogen-(C₁-C₆)-alkyl-(Y)ₚ substituiertes Aryl, Heterocyclyl oder Heteroaryl,
oder
R² und R³ bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten 5- oder 6-gliedrigen, gesättigten, teilweise oder vollständig ungesättigten Ring, der n Heteroatome aus der Gruppe Sauerstoff und Stickstoff enthält,
oder
R² und R³ bilden gemeinsam mit dem sie bindenden Stickstoffatom einen durch v Reste aus der Gruppe Cyano, Nitro, Halogen, (C₁-C₆)-Alkyl-(Y)ₚ und Halogen-(C₁-C₆)-alkyl-(Y)ₚ substituierten Ring aus der Gruppe Benzothiazol, Benzoxazol, Benzopyrazol und Benzopyrrol;
R⁴ bedeutet Wasserstoff, (C₁-C₆)-Alkyl oder (C₁-C₆)-Halogenalkyl, (C₃-C₉)-Cycloalkyl oder (C₃-C₉)-Halogencycloalkyl;
R⁵ bedeutet (C₁-C₆)-Alkyl, Halogen-(C₁-C₆)-alkyl, (C₃-C₉)-Cycloalkyl, (C₃-C₉)-Halogen-cycloalkyl, oder jeweils durch v Reste aus der Gruppe Halogen, Nitro, Cyano, (C₁-C₄)-Alkyl, Halogen-(C₁-C₄)-alkyl, (C₁-C₄)-Alkoxy und Halogen-(C₁-C₄)-alkoxy substituiertes Phenyl;
R⁶ bedeutet Wasserstoff, (C₁-C₆)-Alkyl, Halogen-(C₁-C₆)-alkyl, (C₁-C₆)-Alkylcarbonyl, Halogen-(C₁-C₆)-alkylcarbonyl, (C₁-C₆)-Alkoxycarbonyl, Halogen-(C₁-C₆)-alkoxycarbonyl, (C₁-C₆)-Alkylaminocarbonyl, Halogen-(C₁-C₆)-alkylaminocarbonyl, (C₁-C₆)-Dialkylaminocarbonyl, Halogen-(C₁-C₆)-dialkylaminocarbonyl, (C₁-C₆)-Alkylsulfonyl, Halogen-(C₁-C₆)-alkylsulfonyl, jeweils durch v Reste aus der Gruppe Halogen, Nitro, Cyano, (C₁-C₄)-Alkyl, Halogen-(C₁-C₄)-alkyl, (C₁-C₄)-Alkoxy und Halogen-(C₁-C₄)-alkoxy substituiertes Benzyl, Benzoyl, Benzoylmethyl, Phenoxycarbonyl oder Phenylsulfonyl;
m bedeutet 1 oder 2;
n bedeutet 0, 1 oder 2;
p bedeutet 0 oder 1;
v bedeutet 0, 1, 2 oder 3;
w und x bedeuten jeweils unabhängig voneinander 0,1, 2, 3 oder 4;
wobei w und x nicht gleichzeitig null sein sollen.

2. Verbindungen nach Anspruch 1, worin
R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉-cycloalkyl, (C₁-C₆)-Alkyl-(C₃-C₉)-cycloalkenyl, (C₂-C₆)-Alkenyl-(C₃-C₉)-cycloalkyl, (C₂-C₆)-Alkenyl-(C₃-C₉)-cycloalkenyl, (C₂-C₆)-Alkinyl-(C₃-C₉)-cycloalkyl, (C₂-C₆)-Alkinyl-(C₃-C₉)-cycloalkenyl, geradkettiges oder verzweigtes [O-C(R ⁶)₂]_{w}-[O-C(R⁶)-₂]ₓ-R⁶, (C₁-C₆)-Alkyl-aryl, (C₂-C₆)-Alkenyl-aryl, (C₂-C₆)-Alkinyl-aryl, geradkettiges oder verzweigtes [O-C(R⁶)₂]_{w}-[O-C(R⁶)₂]ₓ-Aryl, wobei die letzten 16 der vorstehend genannten Reste durch v Reste aus der Gruppe Cyano, Nitro und Halogen substituiert sind,
durch v Reste aus der Gruppe Cyano, Nitro, Halogen, (C₁-C₆)-Alkyl-(Y)ₚ und Halogen-(C₁-C₆)-alkyl-(Y)ₚ substituiertes Aryl,
oder
R² und R³ bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten 5- oder 6-gliedrigen, gesättigten, teilweise oder vollständig ungesättigten Ring, der n Heteroatome aus der Gruppe Sauerstoff und Stickstoff enthält,
oder
R² und R³ bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten Ring aus der Gruppe Benzothiazol, Benzoxazol, Benzopyrazol und Benzopyrrol.

3. Verbindungen nach Anspruch 1 oder 2, worin Y für Sauerstoff und R^{1c} für Wasserstoff stehen.

4. Verbindungen nach einem der Ansprüche 1 bis 3, worin
X O oder S(O)ₙ bedeutet;
R^{1a}, R^{1b} unabhängig voneinander F, Cl, Br, CH₃, CH₃S, CH₃O, CH₃SO₂, C₂H₅SO₂, CF₃CH₂SO₂, Cyclopropyl-SO₂, CF₃ oder NO₂ bedeuten;
R², R³ unabhängig voneinander Wasserstoff, (C₁-C₆)-Alkyl, (C₂-C₆)-Alkenyl, (C₂-C₆)-Alkinyl, (C₃-C₉)-Cycloalkyl, (C₁ -C₆)-Alkyl-(C₃-C₉)-cycloalkyl, wobei die letzten 5 genannten Reste mit v Resten aus der Gruppe Cyano, Nitro und Halogen substituiert sind, Aryl, (C₁-C₆)-Alkyl-aryl, wobei die letzten 2 genannten Reste mit v Resten aus der Gruppe Cyano, Nitro, Halogen, (C₁-C₆)-Alkyl-(Y)ₚ und Halo-(C₁-C₆)-alkyl-(Y)ₚ substituiert sind, bedeuten, oder
R² und R³ bilden gemeinsam mit dem sie bindenden Stickstoffatom einen jeweils durch v Reste aus der Gruppe Cyano, Nitro, Halogen, (C₁-C₆)-Alkyl-(Y)ₚ und Halogen-(C₁-C₆)-alkyl-(Y)ₚ substituierten 5- oder 6-gliedrigen, gesättigten, teilweise oder vollständig ungesättigten Ring, der n Heteroatome aus der Gruppe Sauerstoff und Stickstoff enthält,
oder
R² und R³ bilden gemeinsam mit dem sie bindenden Stickstoffatom einen durch v Reste aus der Gruppe Cyano, Nitro, Halogen, (C₁-C₆)-Alkyl-(Y)ₚ und Halogen-(C₁-C₆)-alkyl-(Y)ₚ substituierten Ring aus der Gruppe Benzothiazol, Benzoxazol, Benzopyrazol und Benzopyrrol.

5. Verbindungen nach einem der Ansprüche 1 bis 4, worin X für Sauerstoff steht.

6. Verbindungen nach einem der Ansprüche 1 bis 5, worin
R², R³ unabhängig voneinander Wasserstoff oder (C₁-C₆)-Alkyl bedeuten,
oder
R² und R³ bilden gemeinsam mit dem sie bindenden Stickstoffatom einen Ring aus der Gruppe Morpholin, Pyrrolidin, Piperidin, Pyrrol, Pyrazol und 2,3-Dihydroindol;
R⁴ für Wasserstoff, Methyl oder Cyclopropyl steht.

7. Verbindungen nach einem der Ansprüche 1 bis 6, worin
R⁶ Wasserstoff, (C₁-C₆)-Alkyl, (C₁-C₆)-Alkylcarbonyl, (C₁-C₆)-Alkylsulfonyl, jeweils durch v Reste aus der Gruppe Halogen, Nitro, Cyano, (C₁-C₄)-Alkyl, Halogen-(C₁-C₄)-alkyl, (C₁-C₄)-Alkoxy und Halogen-(C₁-C₄)-alkoxy substituiertes Benzoyl oder Phenylsulfonyl bedeutet.

8. Verbindungen nach einem der Ansprüche 1 bis 7, worin
L CH₂, C(CH₃)H oder CH₂CH₂ bedeutet;
R^{1a}, R^{1b} unabhängig voeinander Cl, Br, NO₂, CH₃, CH₃SO₂ oder C₂H₅SO₂ bedeuten;
R², R³ jeweils Wasserstoff oder (C₁-C₆)-Alkyl bedeuten;
R⁵ Methyl oder Ethyl bedeutet.

9. Herbizide Mittel, **gekennzeichnet durch** einen herbizid wirksamen Gehalt an mindestens einer Verbindung der allgemeinen Formel (I) gemäß einem der Ansprüche 1 bis 8.

10. Herbizide Mittel nach Anspruch 9 in Mischung mit Formulierungshilfsmitteln.

11. Verfahren zur Bekämpfung unerwünschter Pflanzen, **dadurch gekennzeichnet, daß** man eine wirksame Menge mindestens einer Verbindung der allgemeinen Formel (I) nach einem der Ansprüche 1 bis 8 oder eines herbiziden Mittels nach Anspruch 9 oder 10 auf die Pflanzen oder auf den Ort des unerwünschten Pflanzenwachstums appliziert.

12. Verwendung von Verbindungen der allgemeinen Formel (I) nach einem der Ansprüche 1 bis 8 oder von herbiziden Mitteln nach Anspruch 9 oder 10 zur Bekämpfung unerwünschter Pflanzen.

13. Verwendung nach Anspruch 12, **dadurch gekennzeichnet, daß** die Verbindungen der allgemeinen Formel (I) zur Bekämpfung unerwünschter Pflanzen in Kulturen von Nutzpflanzen eingesetzt werden.

14. Verwendung nach Anspruch 13, **dadurch gekennzeichnet, daß** die Nutzpflanzen transgene Nutzpflanzen sind.

## Claims

1. A compound of the formula (I) or salt thereof 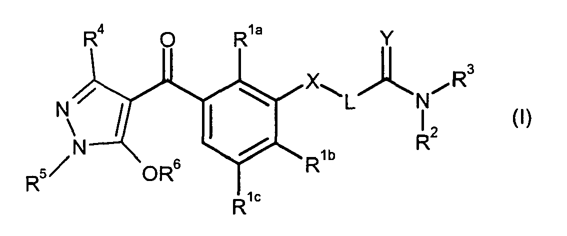 in which the radicals and the indices have the following definitions:
X is O, S(O)ₙ, N-H or N-R²;
L is a straight-chain or branched (C₁-C₆)-alkylene, (C₂-C₆)-alkenylene or (C₂-C₆)alkynylene chain substituted by w radicals from the group consisting of halogen, cyano, and nitro and by v radicals R²;
Y is oxygen or sulfur;
R^{1a}, R^{1b}, R^{1c} independently are each hydrogen, mercapto, nitro, halogen, cyano, thiocyanato,
(C₁-C₆)-alkyl-CO-O, (C₁-C₆)-alkyl-S(O)ₙ-O, (C₁-C₆)-alkyl-S(O)ₘ, (C₁-C₆)-haloalkyl-S(O)ₘ, (C₃-C₇)-cycloalkyl-S(O)ₘ, di-(C₁-C₆)-alkyl-N-SO₂, (C₁-C₆)-alkyl-SO₂-NH, (C₁-C₆)-alkyl-NH-CO, di-(C₁-C₆)-alkyl-N-CO, (C₁-C₆)-alkyl-SO₂-[(C₁-C₆)-alkyl]amino, (C₁-C₆)-alkyl-CO-[(C₁-C₆)-alkyl]amino, (C₁-C₆)-alkyl-O-CH₂, (C₁-C₆)-alkyl-S(O)ₙ-CH₂, (C₁-C₆)-alkyl-NH-CH₂, 1,2,4-triazol-1-yl, 1,2,4-triazol-1-yl-CH₂, or are each (C₁-C₆)-alkyl-(Y)ₚ, (C₂-C₆)-alkenyl-(Y)ₚ, (C₂-C₆)-alkynyl-(Y)ₚ, (C₃-C₉)-cycloalkyl-(Y)ₚ, (C₃-C₉)-cycloalkenyl-(Y)ₚ, (C₁-C₆)-alkyl-(C₃-C₉)-cycloalkyl-(Y)ₚ or (C₁-C₆)-alkyl-(C₃-C₉)-cycloalkenyl-(Y)ₚ each of which is substituted by v radicals from the group consisting of cyano, nitro and halogen;
R², R³ independently are each hydrogen, (C₁-C₆)-alkyl, (C₂-C₆)-alkenyl, (C₂-C₆)-alkynyl, (C₃-C₉)-cycloalkyl, (C₃-C₉)-cycloalkenyl, (C₁-C₆)-alkyl-(C₃-C₉)-cycloalkyl, (C₁-C₆)-alkyl-(C₃-C₉)-cycloalkenyl, (C₂-C₆)-alkenyl-(C₃-C₉)-cycloalkyl, (C₂-C₆)-alkenyl-(C₃-C₉)-cycloalkenyl, (C₂-C₆)-alkynyl-(C₃-C₉)-cycloalkyl, (C₂-C₆)-alkynyl-(C₃-C₉)-cycloalkenyl, straight-chain or branched [O-C(R⁶)₂]_{w}-[O-C(R⁶)-2]ₓ-R⁶, (C₁-C₆)-alkyl-aryl, (C₂-C₆)-alkenyl-aryl, (C₂-C₆)-alkynyl-aryl, straight-chain or branched [O-C(R⁶)₂]_{w}-[O-C(R⁶)₂]ₓ-aryl, the last 16 of the abovementioned radicals being substituted by v radicals from the group consisting of cyano, nitro and halogen,
or are each aryl, heterocyclyl or heteroaryl each substituted by v radicals consisting of the group of cyano, nitro, halogen, (C₁-C₆)-alkyl-(Y)ₚ and halo-(C₁-C₆)-alkyl-(Y)ₚ,
or
R² and R³ together with the nitrogen atom linking them form a 5- or 6-membered saturated, partly unsaturated or fully unsaturated ring which contains n heteroatoms from the group consisting of oxygen and nitrogen and is substituted by v radicals from the group consisting of cyano, nitro, halogen, (C₁-C₆)-alkyl-(Y)ₚ and halo-(C₁-C₆)-alkyl-(Y)ₚ,
or
R² and R³ together with the nitrogen atom linking them form a ring from the group consisting of benzothiazole, benzoxazole, benzopyrazole and benzopyrrole which is substituted by v radicals from the group consisting of cyano, nitro, halogen, (C₁-C₆)-alkyl(Y)ₚ, and halo-(C₁-C₆)-alkyl-(Y)ₚ;
R⁴ is hydrogen, (C₁-C₆)-alkyl or (C₁-C₆)-haloalkyl, (C₃-C₉)-cycloalkyl or (C₃-C₉)-halocycloalkyl;
R⁵ is (C₁-C₆)-alkyl, halo-(C₁-C₆)-alkyl, (C₃-C₉)-cycloalkyl, (C₃-C₉)-halo-cycloalkyl, or is phenyl substituted by v radicals from the group consisting of halogen, nitro, cyano, (C₁-C₄)-alkyl, halo-(C₁-C₄)-alkyl, (C₁-C₄)-alkoxy and halo-(C₁-C₄)-alkoxy;
R⁶ is hydrogen, (C₁-C₆)-alkyl, halo-(C₁-C₆)-alkyl, (C₁-C₆)-alkylcarbonyl, halo-(C₁-C₆)-alkylcarbonyl, (C₁-C₆)-alkoxycarbonyl, halo-(C₁-C₆)-alkoxycarbonyl, (C₁-C₆)-alkylaminocarbonyl, halo-(C₁-C₆)-alkylaminocarbonyl, (C₁-C₆)-dialkylaminocarbonyl, halo-(C₁-C₆)-dialkylaminocarbonyl, (C₁-C₆)-alkylsulfonyl, halo-(C₁-C₆)-alkylsulfonyl, or is benzyl, benzoyl, benzoylmethyl, phenoxycarbonyl or phenylsulfonyl each of which is substituted by v radicals from the group consisting of halogen, nitro, cyano, (C₁-C₄)-alkyl, halo-(C₁-C₄)-alkyl, (C₁-C₄)-alkoxy and halo-(C₁-C₄)-alkoxy;
m is 1 or 2;
n is 0, 1 or 2;
p is 0 or 1 ;
v is 0, 1, 2 or 3;
w and x independently are each 0,1, 2, 3 or 4;
w and x should not both be zero at the same time.

2. A compound as claimed in claim 1, wherein
R², R³ independently are each hydrogen, (C₁-C₆)-alkyl, (C₂-C₆)-alkenyl, (C₂-C₆)-alkynyl, (C₃-C₉)-cycloalkyl, (C₃-C₉)-cycloalkenyl, (C₁-C₆)-alkyl-(C₃-C₉)-cycloalkyl, (C₁-C₆)-alkyl-(C₃-C₉)-cycloalkenyl, (C₂-C₆)-alkenyl-(C₃-C₉)-cycloalkyl, (C₂-C₆)-alkenyl-(C₃-C₉)-cycloalkenyl, (C₂-C₆)-alkynyl-(C₃-C₉)-cycloalkyl, (C₂-C₆)-alkynyl-(C₃-C₉)-cycloalkenyl, straight-chain or branched [O-C(R⁶)₂]_{w}-[O-C(R⁶)-₂]ₓ-R⁶, (C₁-C₆)-alkyl-aryl, (C₂-C₆)-alkenyl-aryl, (C₂-C₆)-alkynyl-aryl, straight-chain or branched [O-C(R⁶)₂]_{w}-[O-C(R⁶)₂]ₓ -aryl, the last 16 of the abovementioned radicals being substituted by v radicals from the group consisting of cyano, nitro, and halogen,
aryl substituted by v radicals from the group consisting of cyano, nitro, halogen, (C₁-C₆)-alkyl-(Y)ₚ and halo-(C₁-C₆)-alkyl-(Y)ₚ
or
R² and R³ together with the nitrogen atom linking them form a 5- or 6-membered saturated, partly unsaturated or fully unsaturated ring which contains n heteroatoms from the group consisting of oxygen and nitrogen and is substituted by v radicals from the group consisting of cyano, nitro, halogen, (C₁-C₆)-alkyl-(Y)ₚ and halo-(C₁-C₆)-alkyl-(Y)ₚ,
or
R² and R³ together with the nitrogen atom linking them form a ring from the group consisting of benzothiazole, benzoxazole, benzopyrazole and benzopyrrole which is substituted by v radicals from the group consisting of cyano, nitro, halogen, (C₁-C₆)-alkyl-(Y)ₚ and halo-(C₁-C₆)-alkyl-(Y)ₚ.

3. A compound as claimed in claim 1 or 2, wherein Y is oxygen and R^{1c} is hydrogen.

4. A compound as claimed in any of claims 1 to 3, wherein
X is O or S(O)ₙ;
R^{1a}, R^{1b} independently are each F, Cl, Br, CH₃, CH₃S, CH₃O, CH₃SO₂, C₂H₅SO₂, CF₃CH₂SO₂, cyclopropyl-SO₂, CF₃ or NO₂;
R², R³ independently are each hydrogen, (C₁-C₆)-alkyl, (C₂-C₆)-alkenyl, (C₂-C₆)-alkynyl, (C₃-C₉)-cycloalkyl, (C₁-C₆)-alkyl-(C₃-C₉)-cycloalkyl, the last 5 radicals being substituted by v radicals from the group consisting of cyano, nitro, and halogen, or are aryl or (C₁-C₆)-alkyl-aryl, the last 2 radicals being substituted by v radicals from the group consisting of cyano, nitro, halogen, (C₁-C₆)-alkyl-(Y)ₚ and halo-(C₁-C₆)-alkyl-(Y)ₚ, or
R² and R³ together with the nitrogen atom linking them form a 5- or 6-membered saturated, partly unsaturated or fully unsaturated ring which contains n heteroatoms from the group consisting of oxygen and nitrogen and is substituted by v radicals from the group consisting of cyano, nitro, halogen, (C₁-C₆)-alkyl-(Y)ₚ and halo-(C₁-C₆)-alkyl-(Y)ₚ,
or
R² and R³ together with the nitrogen atom linking them form a ring from the group consisting of benzothiazole, benzoxazole, benzopyrazole and benzoypyrrole which is substituted by v radicals from the group consisting of cyano, nitro, halogen, (C₁-C₆)-alkyl-(Y)ₚ and halo(C₁-C₆)-alkyl-(Y)ₚ.

5. A compound as claimed in any of claims 1 to 4, wherein X is oxygen.

6. A compound as claimed in any of claims 1 to 5, wherein
R², R³ independently are each hydrogen or (C₁-C₆)-alkyl,
or
R² and R³ together with the nitrogen atom linking them form a ring from the group consisting of morpholine, pyrrolidine, piperidine, pyrrole, pyrazole and 2,3-dihydroindole;
R⁴ is hydrogen, methyl or cyclopropyl.

7. A compound as claimed in any of claims 1 to 6, wherein
R⁶ is hydrogen, (C₁-C₆)-alkyl, (C₁-C₆)-alkylcarbonyl, (C₁-C₆)-alkylsulfonyl, or is benzoyl or phenylsulfonyl each of which is substituted by v radicals from the group consisting of halogen, nitro, cyano, (C₁-C₄)-alkyl, halo-(C₁-C₄)-alkyl, (C₁-C₄)-alkoxy and halo-(C₁-C₄)-alkoxy.

8. A compound as claimed in any of claims 1 to 7, wherein
L is CH₂, C(CH₃)H or CH₂CH₂;
R^{1a}, R^{1b} independently are each Cl, Br, NO₂, CH₃, CH₃SO₂ or C₂H₅SO₂;
R², R³ are each hydrogen or (C₁-C₆)-alkyl;
R⁵ is methyl or ethyl.

9. A herbicidal composition comprising a herbicidally effective amount of at least one compound of the general formula (I) as claimed in any of claims 1 to 8.

10. A herbicidal composition as claimed in claim 9 in a mixture with formulating auxiliaries.

11. A method of controlling unwanted plants, which comprises applying an effective amount of at least one compound of the general formula (I) as claimed in any of claims 1 to 8 or of a herbicidal composition as claimed in claim 9 or 10 to the plants or to the site of the unwanted plant growth.

12. The use of the compound of the general formula (I) as claimed in any of claims 1 to 8 or of a herbicidal composition as claimed in claim 9 or 10 to control unwanted plants.

13. The use as claimed in claim 12, wherein the compound of the general formula (I) is used to control unwanted plants in crops of useful plants.

14. The use as claimed in claim 13, wherein the useful plants are transgenic.

## Revendications

1. Composés de formule (I) ou leurs sels, 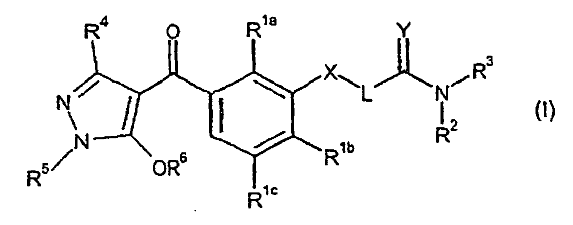 dans laquelle les radicaux et les indices ont les significations suivantes :
X représente O, S(O)ₙ, N-H ou N-R² ;
L représente une chaîne alkylène(en C₁ à C₆), alcénylène(en C₂ à C₆) ou alcynylène(en C₂ à C₆) linéaire ou ramifiée, substituée par w groupes choisis dans le groupe constitué par un atome d'halogène, un groupe cyano et nitro, et par v groupes R² ;
Y représente un atome d'oxygène ou de soufre ;
R^{1a}, R^{1b}, R^{1c} représentent, indépendamment les uns des autres, un atome d'hydrogène, un groupe mercapto, nitro, un atome d'halogène, un groupe cyano, thiocyanato, alkyle(en C₁ à C₆)-CO-O, alkyle(en C₁ à C₆)-S(O)ₙ-O, alkyle (en C₁ à C₆)-S(O)ₘ, halogénoalkyle(en C₁ à C₆)-S(O)ₘ, cycloalkyle(en C₃ à C₇)-S(O)ₘ, di-alkyle (en C₁ à C₆)-N-SO₂, alkyle (en C₁ à C₆)-SO₂-NH, alkyle(en C₁ à C₆)-NH-CO, dialkyle(en C₁ à C₆)-N-CO, alkyle(en C₁ à C₆) -SO₂- [alkyle (en C₁ à C₆)]amino, alkyle(en C₁ à C₆)-CO-[(alkyle en C₁ à C₆)]amino, alkyle(en C₁ à C₆)-O-CH₂, alkyle(en C₁ à C₆)-S(O)ₙ-CH₂, alkyle(en C₁ à C₆)-NH-CH₂, 1,2,4-triazol-1-yle, 1,2,4-triazol-1-yle-CH₂,
un groupe alkyle(en C₁ à C₆)-(Y)ₚ, alcényle(en C₂ à C₆)-(Y)ₚ, alcynyle(en C₂ à C₆)-(Y)ₚ, cycloalkyle(en C₃ à C₉)-(Y)ₚ, cycloalcényle(en C₃ à C₉)-(Y)ₚ, alkyle (en C₁ à C₆)-cycloalkyle(en C₃ à C₉)-(Y)ₚ ou alkyle(en C₁ à C₆)-cycloalcényle(en C₃ à C₉)-(Y)ₚ, chacun substitué par v groupes choisis dans le groupe constitué par un groupe cyano, nitro et un atome d'halogène ;
R², R³ représentent, indépendamment l'un de l'autre, un atome d'hydrogène, un groupe alkyle(en C₁ à C₆), alcényle (en C₂ à C₆), alcynyle (en C₂ à C₆), cycloalkyle(en C₃ à C₉), cycloalcényle (en C₃ à C₉), alkyle(en C₁ à C₆)-cycloalkyle(en C₃ à C₉), alkyle(en C₁ à C₆)-cycloalcényle(en C₃ à C₉), alcényle(en C₂ à C₆)-cycloalkyle (en C₃ à C₉), alcényle(en C₂ à C₆)-cycloalcényle(en C₃ à C₉), alcynyle(en C₂ à C₆)-cycloalkyle(en C₃ à C₉), alcynyle(en C₂ à C₆)-cycloalcényle(en C₃ à C₉), un groupe [O-C(R⁶)₂)_{w}-[O-C(R⁶)-₂]ₓ-R⁶ linéaire ou ramifié, alkyle (en C₁ à C₆)-aryle, alcényle (en C₂ à C₆)-aryle, alcynyle(en C₂ à C₆)-aryle, un groupe [O-C(R⁶)₂]_{w}-[O-C(R⁶)₂]ₓ-aryle linéaire ou ramifié, les 16 derniers groupes mentionnés étant substitués par v groupes choisis dans le groupe constitué par un groupe cyano, nitro et un atome d'halogène, un groupe aryle, hétérocyclyle ou hétéroaryle, chacun substitué par v groupes choisis dans le groupe constitué par un groupe cyano, nitro, un atome d'halogène, un groupe alkyle (en C₁ à C₆)-(Y)ₚ et un groupe halogénoalkyle (en C₁ à C₆)-(Y)ₚ,
ou
R² et R³ forment, conjointement avec l'atome d'azote auquel ils sont liés, un cycle à 5 ou 6 éléments, saturé, partiellement ou entièrement insaturé, qui contient n hétéroatomes choisis dans le groupe constitué par un atome d'oxygène et un atome d'azote, substitué par v groupes choisis dans le groupe constitué par un groupe cyano, nitro, un atome d'halogène, un groupe alkyle (en C₁ à C₆)-(Y)ₚ et halogénoalkyle (en C₁ à C₆)-(Y)ₚ,
ou
R² et R³ forment, conjointement avec l'atome d'azote auquel ils sont liés, un cycle choisi dans le groupe constitué par un groupe benzothiazole, benzoxazole, benzopyrazole et benzopyrrole, substitué par v groupes choisis dans le groupe constitué par un groupe cyano, nitro, un atome d'halogène, un groupe alkyle(en C₁ à C₆)-(Y)ₚ et un groupe halogénoalkyle(en C₁ à C₆)-(Y)ₚ ;
R⁴ représente un atome d'hydrogène, un groupe alkyle(en C₁ à C₆) ou halogénoalkyle(en C₁ à C₆), cycloalkyle(en C₃ à C₉) ou halogénocycloalkyle(en C₃ à C₉) ;
R⁵ représente un groupe alkyle(en C₁ à C₆), halogénoalkyle(en C₁ à C₆), cycloalkyle(en C₃ à C₉), halogénocycloalkyle(en C₃ à C₉), ou un groupe phényle substitué par v groupes choisis dans le groupe constitué par un atome d'halogène, un groupe nitro, cyano, alkyle(en C₁ à C₄), halogénoalkyle(en C₁ à C₄), alcoxy(en C₁ à C₄) et halogénoalcoxy(en C₁ à C₄) ;
R⁶ représente un atome d'hydrogène, un groupe alkyle(en C₁ à C₆), halogénoalkyle(en C₁ à C₆), alkyle(en C₁ à C₆)-carbonyle, halogénoalkyle(en C₁ à C₆)-carbonyle, alcoxy(en C₁ à C₆)-carbonyle, halogénoalcoxy(en C₁ à C₆)-carbonyle, alkyle(en C₁ à C₆)-aminocarbonyle, halogénoalkyle(en C₁ à C₆)-aminocarbonyle, dialkyle(en C₁ à C₆)-aminocarbonyle, halogénodialkyle(en C₁ à C₆)-carbonyle, alkyle(en C₁ à C₆)-sulfonyle, halogénoalkyle(en C₁ à C₆)-sulfonyle,
un groupe benzyle, benzoyle, benzoylméthyle, phénoxycarbonyle ou phénylsulfonyle, chacun substitué par v groupes choisis dans le groupe constitué par un atome d'halogène, un groupe nitro, cyano, alkyle(en C₁ à C₄), halogénoalkyle(en C₁ à C₄), alcoxy(en C₁ à C₄) et halogénoalcoxy(en C₁ à C₄);
m vaut 1 ou 2 ;
n vaut 0, 1 ou 2 ;
p vaut 0 ou 1 ;
v vaut 0, 1, 2 ou 3 ;
w et x valent chacun indépendamment l'un de l'autre 0, 1, 2, 3 ou 4 ; w et x ne pouvant pas valoir en même temps zéro.

2. Composés selon la revendication 1, dans lesquels
R², R³ représentent, indépendamment l'un de l'autre, un atome d'hydrogène, un groupe alkyle(en C₁ à C₆), alcényle(en C₂ à C₆), alcynyle(en C₂ à C₆), cycloalkyle(en C₃ à C₉), cycloalcényle(en C₃ à C₉), alkyle (en C₁ à C₆)-cycloalkyle(en C₃ à C₉), alkyle(en C₁ à C₆)-cycloalcényle(en C₃ à C₉), alcényle(en C₂ à C₆)-cycloalkyle(en C₃ à C₉), alcényle(en C₂ à C₆)-cycloalcényle(en C₃ à C₉), alcynyle(en C₂ à C₆)-cycloalkyle(en C₃ à C₉), alcynyle (en C₂ à C₆)-cycloalcényle (en C₃ à C₉), un groupe [O-C(R⁶)₂)]_{w}-[O-C(R⁶)-₂]ₓ-R⁶ linéaire ou ramifié, alkyle(en C₁ à C₆) -aryle, alcényle(en C₂ à C₆)-aryle, alcynyle(en C₂ à C₆)-aryle, un groupe [O-C(R⁶)₂]_{w}-[O-C(R⁶)₂]ₓ-aryle linéaire ou ramifié, les 16 derniers groupes mentionnés étant substitués par v groupes choisis dans le groupe constitué par un groupe cyano, nitro et un atome d'halogène, un groupe aryle substitué par v groupes choisis dans le groupe constitué par un groupe cyano, nitro, un atome d'halogène, un groupe alkyle(en C₁ à C₆)-(Y)ₚ et halogénoalkyle(en C₁ à C₆)-(Y)ₚ,
ou
R² et R³ forment, conjointement avec l'atome d'azote auquel ils sont liés, un cycle à 5 ou 6 éléments, saturé, partiellement ou entièrement insaturé, qui contient n hétéroatomes choisis dans le groupe constitué par un atome d'oxygène et un atome d'azote, substitué par v groupes choisis dans le groupe constitué par un groupe cyano, nitro, un atome d'halogène, un groupe alkyle(en C₁ à C₆)-(Y)ₚ et halogénoalkyle(en C₁ à C₆)-(Y)ₚ,
ou
R² et R³ forment, conjointement avec l'atome d'azote auquel ils sont liés, un cycle choisi dans le groupe constitué par un groupe benzothiazole, benzoxazole, benzopyrazole et benzopyrrole, substitué par v groupes choisis dans le groupe constitué par un groupe cyano, nitro, un atome d'halogène, un groupe alkyle(en C₁ à C₆)-(Y)ₚ et un groupe halogénoalkyle(en C₁ à C₆)-(Y)ₚ.

3. Composés selon la revendication 1 ou 2, dans lesquels Y représente un atome d'oxygène et R^{1c} représente un atome d'hydrogène.

4. Composés selon l'une quelconque des revendications 1 à 3, dans lesquels
X représente O ou S(O)ₙ ;
R^{1a}, R^{1b} représentent, indépendamment l'un de l'autre F, Cl, Br, CH₃, CH₃S, CH₃O, CH₃SO₂, C₂H₅SO₂, CF₃CH₂SO₂, cyclopropyle-SO₂, CF₃ ou NO₂ ;
R², R³ représentent, indépendamment l'un de l'autre, un atome d'hydrogène, un groupe alkyle(en C₁ à C₆), alcényle(en C₂ à C₆), alcynyle(en C₂ à C₆), cycloalkyle(en C₃ à C₆), alkyle(en C₁ à C₆)-cycloalkyle(en C₃ à Cg), les 5 derniers groupes mentionnés étant substitués par v groupes choisis dans le groupe formé par un groupe cyano, nitro et un atome d'halogène, un groupe aryle, alkyle(en C₁ à C₆)-aryle, les 2 derniers groupes mentionnés étant substitués par v groupes choisis dans le groupe formé par un groupe cyano, nitro, un atome d'halogène, un groupe alkyle(en C₁ à C₆)-(Y)ₚ et halogénoalkyle(en C₁ à C₆)-(Y)ₚ,
ou
R² et R³ forment, conjointement avec l'atome d'azote auquel ils sont liés, un cycle à 5 ou 6 éléments, saturé, partiellement ou entièrement insaturé, qui contient n hétéroatomes choisis dans le groupe constitué par un atome d'oxygène et un atome d'azote, substitué par v groupes choisis dans le groupe constitué par un groupe cyano, nitro, un atome d'halogène, un groupe alkyle (en C₁ à C₆)-(Y)ₚ et halogénoalkyle(en C₁ à C₆)-(Y)ₚ,
ou
R² et R³ forment, conjointement avec l'atome d'azote auquel ils sont liés, un cycle choisi dans le groupe constitué par un groupe benzothiazole, benzoxazole, benzopyrazole et benzopyrrole, substitué par v groupes choisis dans le groupe constitué par un groupe cyano, nitro, un atome d'halogène, un groupe alkyle(en C₁ à C₆)-(Y)ₚ et un groupe halogénoalkyle (en C₁ à C₆)-(Y)ₚ.

5. Composés selon l'une quelconque des revendications 1 à 4, dans lesquels X représente un atome d'oxygène.

6. Composés selon l'une quelconque des revendications 1 à 5, dans lesquels
R², R³ représentent, indépendamment l'un de l'autre, un atome d'hydrogène ou un groupe alkyle(en C₁ à C₆),
ou
R² et R³ forment, conjointement avec l'atome d'azote auquel ils sont liés, un cycle choisi dans le groupe constitué par la morpholine, la pyrrolidine, la pipéridine, le pyrrol, le pyrazole et le 2,3-dihydroindole ;
R⁴ représente un atome d'hydrogène, un groupe méthyle ou cyclopropyle.

7. Composés selon l'une quelconque des revendications 1 à 6, dans lesquels
R⁶ représente un atome d'hydrogène, un groupe alkyle(en C₁ à C₆), alkyle(en C₁ à C₆)-carbonyle, alkyle(en C₁ à C₆)-sulfonyle, un groupe benzoyle ou phénylsulfonyle, chacun substitué par v groupes choisis dans le groupe constitué par un atome d'halogène, un groupe nitro, cyano, alkyle(en C₁ à C₄), halogénoalkyle(en C₁ à C₄), alcoxy(en C₁ à C₄) et halogénoalcoxy(en C₁ à C₄).

8. Composés selon l'une quelconque des revendications 1 à 7, dans lesquels
L représente CH₂, C(CH₃)H ou CH₂CH₂ ;
R^{1a}, R^{1b} représentent, indépendamment l'un de l'autre, Cl, Br, NO₂, CH₃, CH₃SO₂ ou C₂H₅SO₂ ;
R², R³ représentent chacun un atome d'hydrogène, ou un groupe alkyle en C₁ à C₆ ;
R⁵ représente un groupe méthyle ou éthyle.

9. Agents herbicides, **caractérisés par** une teneur efficace sur le plan herbicide en au moins un composé de formule générale (I) selon l'une quelconque des revendications 1 à 8.

10. Agents herbicides selon la revendication 9 en mélange avec des adjuvants de formulation.

11. Procédé de lutte contre les plantes adventices, **caractérisé en ce que** l'on applique une quantité efficace d'au moins un composé de formule générale (I) selon l'une quelconque des revendications 1 à 8 ou d'un agent herbicide selon la revendication 9 ou 10 sur les plantes ou sur le site où se développent les plantes adventices.

12. Utilisation de composés de formule générale (I) selon l'une quelconque des revendications 1 à 8 ou d'agents herbicides selon la revendication 9 ou 10 pour lutter contre les plantes adventices.

13. Utilisation selon la revendication 12, **caractérisée en ce que** les composés de formule générale (I) sont mis en oeuvre pour lutter contre les plantes adventices dans des cultures de plantes utiles.

14. Utilisation selon la revendication 13, **caractérisée en ce que** les plantes utiles sont des plantes utiles transgéniques.
